# EUROPEAN PATENT SPECIFICATION

(11) **EP 1 109 570 B1**
(45) Date of publication and mention of the grant of the patent: **19.10.2005**
(21) Application number: 99968216.4
(22) Date of filing: 31.08.1999
(51) Int. Cl.: A61K 38/17, A61K 39/395, C12N 5/06, A61P 37/00, A61P 19/02, A61P 27/02, A61P 1/04

(54) **MODULATION OF MEMORY EFFECTOR T-CELLS WITH A CD2 BINDING AGENT**
MODULIERUNG VON GEDÄCHTNIS-EFFECTOR T-ZELLEN UNTER VERWENDUNG EINES CD2-BINDUNGSAGENTES
MODULATION DE CELLULES T EFFECTRICES DE MEMOIRE PAR DES LIGANDS DE CD2

(30) Priority: 31.08.1998 US 98456 P
(43) Date of publication of application: 27.06.2001
(73) Proprietor: BIOGEN, INC., Cambridge Massachusetts 02142 (US)
(72) Inventor: MAGILAVY, Daniel, Cambridge, MA 02140 (US)
(74) Representative: Vossius & Partner
(86) International application number: PCT/US1999/020026
(87) International publication number: WO 2000/012113

(56) References cited:
- EP-A- 0 503 648
- WO-A-93/06852
- N. DAMLE ET AL.: "Stimulation of cloned human T lymphocytes via the CD3 or CD28 molecules induces enhancement in vascular endothelial permeability to macromolecules with participation of type-1 and type-2 intercellular adhesion pathways." EUROPEAN JOURNAL OF IMMUNOLOGY, vol. 20, no. 9, September 1990 (1990-09), pages 1995-2003, XP000892124 Weinheim, Germany
- K. YAMASHITA ET AL.: "A multimeric form of soluble recombinant sheep LFA-3 (CD58) inhibits human T-cell proliferation." IMMUNOLOGY, vol. 92, no. 1, September 1997 (1997-09), pages 39-44, XP002135989 Oxford, GB
- X. PENG ET AL.: "Ligation of CD2 provides a strong helper signal for the production of the type 2 cytokines interleukin-4 and -5 by memory T cells." CELLULAR IMMUNOLOGY, vol. 181, no. 1, 10 October 1997 (1997-10-10), pages 76-85, XP000892106 New York, NY, USA
- J. HOFFMANN ET AL.: "Initiation and perpetuation of rat adjuvant arthritis is inhibited by the anti-CD2 monoclonal antibody (mAb) OX34." ANNALS OF RHEUMATIC DISEASES, vol. 56, no. 12, December 1997 (1997-12), pages 716-722, XP000892112 London, GB
- G. MAJEAU ET AL.: "Mechanism of lymphocyte function-associated molecule 3-Ig fusion proteins inhibition of T cell responses." THE JOURNAL OF IMMUNOLOGY, vol. 152, no. 6, 15 March 1994 (1994-03-15), pages 2753-2767, XP002135990 Baltimore, MD, USA
- Y. KASAHARA ET AL.: "Role of interleukin 6 for differential responsiveness of naive and memory CD4+ T cells in CD2-mediated activation." THE JOURNAL OF EXPERIMENTAL MEDICINE, vol. 172, no. 5, 1 November 1990 (1990-11-01), pages 1419-1424, XP000892122 New York, NY, USA
- P. HOLLSBERG ET AL.: "Increased protein kinase C activity in human memory T cells." CELLULAR IMMUNOLOGY, vol. 149, no. 1, June 1993 (1993-06), pages 170-179, XP000892120 New York, NY, USA
- D. MAGILAVY ET AL.: "Pharmacodynamic effects of LFA3TIP (Amevive) in patients with chronic plaque psoriasis (CPP): Selective modulation of CD45RO+ lymphocytes." JOURNAL OF INVESTIGATIVE DERMATOLOGY, vol. 112, no. 4, April 1999 (1999-04), page 609 XP002136039 Baltimore, MD, USA

## Description

### TECHNICAL FIELD OF THE INVENTION

This invention relates to the use of inhibitors of the CD2/LFA-3 interaction in treating conditions characterized by the presence of activated T cells in mammals, including humans. Such conditions include inflammatory bowel diseases, psoriatic arthritis, rheumatoid arthritis, and multiple sclerosis.

### BACKGROUND OF THE INVENTION

Antigen presenting cells (APC) express a high density of Class II major histocompatibility complex (MHC) antigen on the cell surface. MHC Class II molecules bind peptides derived from endocytosed antigen and are recognized primarily by helper T lymphocytes. The T cell receptor on T cells recognizes antigen as a peptide fragment bound to the cell-surface molecules encoded by the MHC (Springer, "Adhesion Receptors of the Immune System", Nature, 346, pp. 425-27 (1990)).

There are many interactions between molecules expressed on the surface of such APC and the surface of T cells, in addition to the T cell receptor/MHC interaction. These surface molecules, often referred to as adhesion molecules, participate in a number of functions including cellular adhesion, antigen recognition,
co-stimulatory signaling in T cell activation and stimulation of effectors of T cell cytotoxicity ("Adhesion Molecules in Diagnosis and Treatment of Inflammatory Diseases", The Lancet, 336, pp. 1351-52 (1990)). Such cell adhesion appears to be involved in activation of T cell proliferation in the generation of an immune response (Hughes et al., "The Endothelial Cell as a Regulator of T-cell Function", Immunol, Rev., 117, pp. 85-102 (1990)).

One way in which T cells are activated is by binding of their antigen specific T cell receptors to peptide-MHC complexes on the surface of antigen presenting cells such as macrophages. T cell activation stimulates proliferation and differentiation of two types of functional T cells: helper cells, which promote the proliferation and maturation of antibody producing B lymphocytes, and killer cells, which lyse target cells (Bierer et al., "A Monoclonal Antibody to LFA-3, the CD2 Ligand, Specifically Immobilizes Major Histocompatibility Complex Proteins", Eur, J. Immunol. 19: pp. 661-65 (1989); Springer "Adhesion Receptors of the Immune System", Nature, 346, pp. 425-34 (1990)).

There are numerous medical conditions characterized by abnormal immune responses and many of these are, in particular, characterized by increased T cell activation. T cells play a major role in the immune response by interacting with target and antigen presenting cells. For example, T cell-mediated killing of target cells is a multi-step process involving, initially, adhesion of cytolytic T cells (the effector cells) to target cells. Also, helper T cells help initiate the immune response by adhesion to antigen presenting cells.

These interactions of T cells with target and antigen presenting cells are highly specific and depend on the recognition of an antigen on the surface of a target or APC by one of the many specific antigen receptors on the surface of T cells.

The receptor-antigen interaction of T cells and other cells is also facilitated by various T cell surface proteins, e.g., the antigen-receptor complex CD3 and accessory adhesion molecules such as CD4, LFA-1, CD8, and CD2. It is also facilitated by accessory adhesion molecules, such as LFA-3, ICAM- I and MHC, that are expressed on the surface of the target or antigen presenting cells. For example, LFA- I and its counter receptor ICAM- I or ICAM-2, as well as CD2 and its counter receptor LFA-3 have been implicated in cellular adhesion and T cell activation. It is known that the LFA-I/ICAM and CD2/LFA-3 interactions are independent. A number of other molecules present on resting T cells have also been implicated in T cell adhesion, including E2 (MIC2), VLA-4 (CD49d), CD44 (Hermes, Pgp-1, ECMRIII), and H19 (N4) (see Makgoba et al., "The CD2-LFA-3 and LFA-1-ICAM Pathways: Relevance to T-cell Recognition", Immunol, Today, 10, pp. 417-22 (1989)).

The interaction between CD2 and LFA-3 remains poorly understood with respect to activation of T cell activity. Recent studies have suggested that there is a specific interaction between CD2 (a T cell adhesion molecule) and LFA-3 (a target cell and APC adhesion molecule) which mediates T cell adhesion to the target or antigen presenting cells. This cell-cell adhesion has been implicated in the initiation of T cell functional responses (Dustin et al., "Purified Lymphocyte Function Associated Antigen 3 Binds to CD2 and Mediates T Lymphocyte Adhesion," J. Ex-p. Med., 165, pp. 677-92 (1987); Springer et al., "The Lymphocyte Function-associated LFA-1, CD2, and LFA-3 Molecules: Cell Adhesion Receptors of the Immune System", Ann. Rev. Immunol., 5, pp. 223-52 (1987)).

LFA-3, which is found on the surface of a wide variety of cells, including human erythrocytes, has become the subject of a considerable amount of study to further elucidate its role in various T cell interactions (see, e.g., Krensky et al., "The Functional Significance, Distribution, and Structure of LFA-1, LFA-2, and LFA-3: Cell Surface Antigen Associated with CTL-Target Interactions", J. Immunol., 131 (2), pp. 611-16 (1983); Shaw et al., "Two Antigen-Independent Adhesion Pathways Used by Human Cytotoxic T-cell Clones", Nature, 323, pp. 262-64 (1986)).

Two natural forms of LFA-3 have been identified. One form of LFA-3 ("transmembrane LFA-3") is anchored in the cell membrane by a transmembrane hydrophobic domain. cDNA encoding this form of LFA-3 has been cloned and sequenced (see, e.g., Wallner et al., "Primary Structure of Lymphocyte Function-Associated Antigen-3 (LFA-3)", J. Exp. Med., 166, pp. 923-32 (1987)). Another form of LFA-3 is anchored to the cell membrane via a covalent linkage to phosphatidylinositol ("PI")-containing glycolipid. This latter form has been designated "PI-linked LFA-3", and CDNA encoding this form of LFA-3 has also been cloned and sequenced (Wallner et al., PCT Pub. WO 90/02181).

The human CD2 molecule is a 50 kD surface glycoprotein expressed on >95% of thymocytes and virtually all peripheral T lymphocytes. Biochemical analyses using specific monoclonal antibodies have suggested that CD2 is T lineage-specific and exists on the cell surface in several differentially glycosylated forms (Howard et al., "A Human T Lymphocyte Differentiation Marker Defined by Monoclonal Antibodies that Block E-Rosette Formation", J. Immunol., 126, pp. 2117-22 (1981); Brown et al., in Leukocyte Typing III, ed. McMichael, Oxford University Press, pp. 110-12 (1987); Sayre et al., "Molecular Cloning and Expression of T11 cDNAs Reveals a Receptor-Like Structure on Human T Lymphocytes", Proc, Natl, Acad, Sci, USA, 84, pp. 2941-45 (1987)).

The sequence of a human CD2 gene has been reported (Seed and Aruffo, "Molecular Cloning of the CD2 Antigen, the T-cell Erythrocyte Receptor, by a Rapid Immunoselection Procedure", Proc, Natl. Acad, Sci, USA, 84, pp. 3365-69 (1987); Sayre et al., "Molecular Cloning and Expression of T11 cDNAs Reveal a Receptor-like Structure on Human T Lymphocytes", Proc. Natl. Acad, Sci, USA, 84, pp. 2941-45 (1987)). CD2 cDNA clones predict a cleaved signal peptide of 24 amino acid residues, an extracellular segment of 185 residues, a transmembrane domain of 25 residues and a cytoplasmic region of 117 residues (Sayre et al., supra (1987); Sewell et al., "Molecular Cloning of the Human T-Lymphocyte Surface CD2 (Tl 1) Antigen", Proc. Natl. Acad. Sci. USA, 83, pp. 8718-22 (1986); Seed and Aruffo, supra (1987); Clayton et al., Eur, J. Immun., 17, pp. 1367-70 (1987)). Soluble CD2 polypeptides having an LFA-3 binding domain have been reported (PCT Publn. WO 90/08187). Monoclonal antibodies to CD2, for example TS2/18, T11₁, T11₂, T11₃, and to LFA3, for example TS2/9, have also been reported (see, e.g., Hughes et al., "Tbe Endothelial Cell as a Regulator of T-Cell Function", Immunol, Reviews, 117, pp. 85-102 (1990); Meuer, "An Alternative Pathway of T-Cell Activation: A Functional Role for the 50 kD T11 Sheep Erythrocyte Receptor Protein", Cell, 36, pp. 897-906 (1984)).

Binding of the antigen specific T cell receptors to peptide-MHC complexes on the surface of antigen presenting cells is also involved in the formation of so-called "memory" cells which are generally formed during the adaptive immune response requiring contact with antigen and expansion of antigen-specific memory cells. T-cell memory is probably dependent upon repeated stimulation by antigen. Memory T-cells augment their binding avidity for the antigen-presenting cell through increased expression of CD2, LFA-3 and other accessory adhesion molecules. Indeed, an important phenotypic change in the isoform of the leukocyte antigen CD45R allows a distinction to be made between naive T cells which express CD45R and memory T-cells which react with the lower molecular weight form CD45RO.

The realization that most, if not all, memory cells are subject to repeated bombardment from antigen makes it likely that most of the features associated with the CD45RO subset are in fact manifestations of effector T cells. There is a growing and provocative literature describing the association of CD45RO memory effector T cells with the presence of various inflammatory diseases such as inflammatory bowel diseases, including Crohn's disease and ulcerative colitis, (Horiuchi et al., J. Japan. Soc. Colo. Proctol., 45: 391-393 (1992)); psoriatic arthritis (Veale et al., Ann. Rheum. Dis. 53: 450-454 (1994); rheumatoid arthritis (Muller et al., Autoimmunity 14: 37-43 (1992); uveitis (Ohta et al., Curr. Eye Res., 15: 299-306 (1996), nephritis (Rodruguez-Poerez et al., Am. J. Nephrol., 15: 386-391 (1995); and multiple sclerosis (Crucian et al., Clin. Diag. Lab. Immunol., 2: 249-252 (1995), Lehmann et al., Clin. Immunol. Immunopathol., 85: 202-209 (L997), Muller et al., Autoimmunity 14: 37-43 (1992), Qin et al., J. Clin. Immunol., 13: 152-161 (1993).

Because of the possible role of these memory effector T lymphocytes in pathogenesis, the need exists for improved methods of modulating these cells in patients with these conditions.

### SUMMARY OF THE INVENTION

The present invention provides the use of a CD2 binding agent for preventing or treating conditions characterized by infiltration of memory effector T lymphocytes in an organ of the mammal. The CD2 binding agent is capable of modulating the number and/or distribution of memory effector T lymphocytes. The agents of this invention are superior to previously available therapies for these conditions for many reasons, including the fact that the agents are more selective, thus less immunosuppressive than pre-existing therapies with less general toxicity.

Materials that can be used in accordance with the method of the present invention include a molecule that is a CD2 binding agent. This includes molecules or other chemical entities that modulate the CD2/LFA-3 interaction. The agent is selected from the group consisting of anti-LFA-3 antibody homologs, soluble LFA-3 polypeptides, soluble CD2 polypeptides, CD2 or LFA-3 mimetic agents (such as small organic molecules) and derivatives thereof.

One aspect of the invention is the selective modulation of memory effector T lymphocytes in a subject having a medical condition, comprising the use of an effective amount of a CD2 binding agent. The memory T lymphocytes may infiltrate a target organ in the subject. Preferably, the memory effector T lymphocytes are CD45RO T lymphocytes. The CD2 binding agent is selected from the group consisting of anti-LFA-3 antibody homologs, soluble LFA-3 polypeptides, soluble CD2 polypeptides, CD2 mimetic agents, and LFA-3 mimetic agents. Another aspect of the invention involves treating a condition in a subject characterized by implication of memory effector T lymphocytes in pathogenesis, including the use of an effective amount of a CD2 binding agent. The condition is selected from the group consisting of psoriatic arthritis, rheumatoid arthritis, multiple sclerosis, atopic dermatitis, uveitis, inflammatory bowel disease, Crohn's disease, ulcerative colitis, and cutaneous T cell lymphoma.

A further aspect of the invention is the modulation of memory effector T lymphocytes in a mammal, wherein the mammal is characterized by having the presence of infiltrating memory effector T lymphocytes in an organ of the mammal, comprising the use of an amount of a CD2 binding agent sufficient to selectively modulate memory effector T lymphocytes in said organ. The mammal suffers from a condition selected from the group consisting of psoriatic arthritis, rheumatoid arthritis, multiple sclerosis, atopic dermatitis, uveitis, inflammatory bowel disease, Crohn's disease, ulcerative colitis, and cutaneous T cell lymphoma.

Compositions of the invention comprise a population of memory effector T lymphocytes obtainable from a mammal having a condition characterized by the presence of infiltrating memory effector T lymphocytes in an organ of the mammal, wherein the population is in combination with a CD2 binding agent.

### BRIEF DESCRIPTION OF THE DRAWINGS

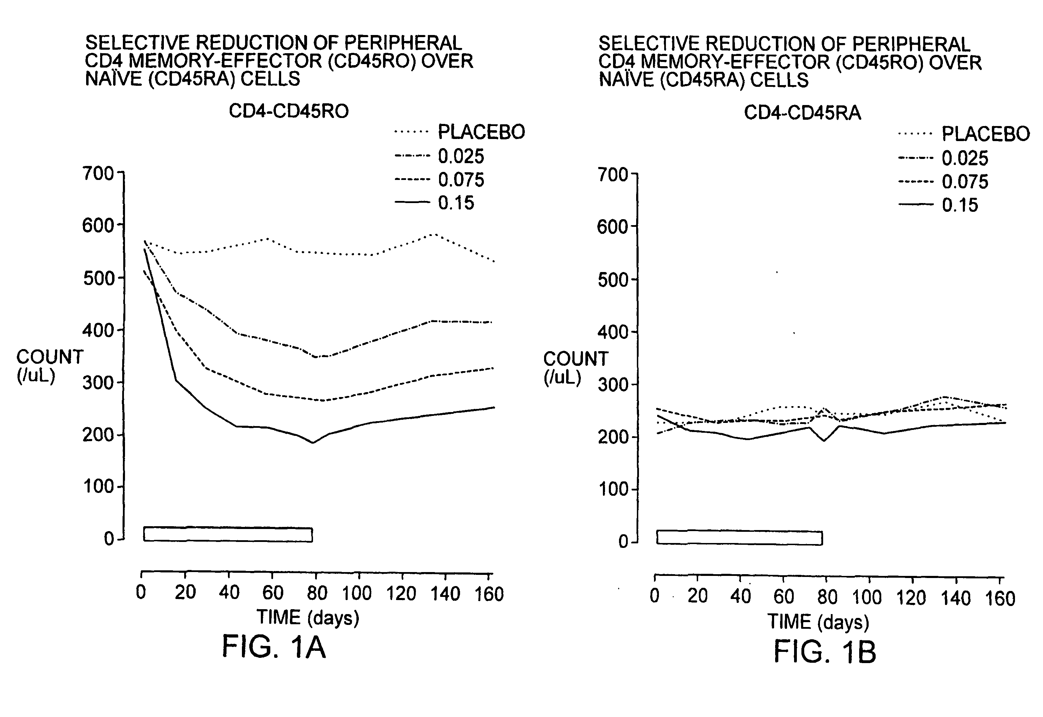

Figure 1 is a graph showing the selective reduction of memory effector T cells relative to naïve T cells in patients with psoriasis receiving treatment with the CD2 binding agent, LFA3-TIP. The horizontal bar above the X axis represents the duration of treatment, which was stopped at approximately 80 days. The curves represent the mean lymphocyte counts from the 57 patients in each treatment group. There are statistically significant treatment and temporal effects with regard to the memory T cells (CD 45 RO+) but not with regard to the naive T cells (CD45 RA+). T cell counts in the various CD2 binding agent treatments show a decrease, approaching some relatively constant value, during the 80 day adminstration period. After treatment was terminated, the memory effector T cells count began to return.

### DETAILED DESCRIPTION OF THE INVENTION

### Definitions

As used herein, "effector T lymphocyte", "effector T cells", "memory effector T lymphocyte/cell" and similar terms are used interchangeably and mean T lymphocytes that have already been exposed to antigen (i.e., those that are not "naive"). More specifically, the terms mean CD45R0 T lymphocytes, as opposed to "naive" CD45RA T lymphocytes.

As used herein, "CD2" means a CD2 polypeptide that binds to a naturally occurring LFA-3 polypeptide and which is encoded by (a) a naturally occurring mammalian CD2 DNA sequence (e.g., SEQ ID NO:5); (b) a DNA sequence degenerate to a naturally occurring CD2 DNA sequence; or (c) a DNA sequence that hybridizes to one of the foregoing DNA sequences under conditions equivalent to about 20 degrees C to 27 degrees C below Tm and 1M sodium chloride.

As used herein, " LFA-3 " means an LFA-3 polypeptide that binds to a naturally occurring CD2 polypeptide and which is encoded by (a) a naturally occurring mammalian LFA-3 DNA sequence (e.g., SEQ ID NO: 1 or SEQ ID NO:3); (b) a DNA sequence degenerate to a naturally occurring LFA-3 DNA sequence; or (c) a DNA sequence that hybridizes to one of the foregoing DNA sequences under standard hybridization conditions, as defined below.

As used herein, a "CD2 binding agent" is a molecule, or compound, or other chemical entity that modulates the CD2/LFA3 interaction and/or modulates CD2 signaling. The term includes anti-LFA-3 antibody homologs, soluble LFA-3 polypeptides, soluble CD2 polypeptides, CD2 or LFA-3 mimetic agents and derivatives thereof. The term "modulates" means that the CD2 binding agent alters (preferably decreases) the intensity of CD2 signaling and/or interferes with the ability of CD2 to bind to LFA3, although increases in such intensity and/or ability to bind are not excluded from the definition. CD2 binding agents also "modulate" memory effector T lymphocytes (i.e., CD45RO) and in this context the term "modulate" means that the binding agents will alter the number and/or distribution of memory effector T lymphocytes in a subject receiving the CD2 binding agent(s). In preferred methods, the binding agent(s) will "selectively" modulate such memory effector T lymphocytes, meaning that the memory effector T lymphocytes are modulated as compared to naïve T lymphocytes (e.g., CD45RA T lymphocytes).

As used herein, a "soluble LFA-3 polypeptide" or a "soluble CD2 polypeptide" is an LFA-3 or CD2 polypeptide incapable of anchoring itself in a membrane. Such soluble polypeptides include, for example, CD2 and LFA-3 polypeptides that lack a sufficient portion of their membrane spanning domain to anchor the polypeptide or are modified such that the membrane spanning domain is non-functional. As used herein soluble LFA-3 polypeptides include full-length or truncated (e.g., with internal deletions) PI-linked LFA-3.

As used herein, an "antibody homolog" is a protein comprising one or more polypeptides selected from immunoglobulin light chains, immunoglobulin heavy chains and antigen-binding fragments thereof which are capable of binding to one or more antigens. The component polypeptides of an antibody homolog composed of more than one polypeptide may optionally be disulfide-bound or otherwise covalently crosslinked. Accordingly, antibody homologs include intact immunoglobulins of types IgA, IgG, IgE, IgD, IgM (as well as subtypes thereof), wherein the light chains of the immunoglobulin may be of types kappa or lambda. Antibody homologs also include portions of intact immunoglobulins that retain antigen-binding specificity, for example, Fab fragments, Fab^{r} fragments, F(ab')2 fragments, F(v) fragments, heavy chain monomers or dimers, light chain monomers or dimers, dimers consisting of one heavy and one light chain, and the like.

As used herein, a "humanized recombinant antibody homolog" is an antibody homolog, produced by recombinant DNA technology, in which some or all of the amino acids of a human immunoglobulin light or heavy chain that are not required for antigen binding have been substituted for the corresponding amino acids from a non human mammalian immunoglobulin light or heavy chain.
As used herein, a "chimeric recombinant antibody homolog" is an antibody homolog, produced by recombinant DNA technology, in which all or part of the hinge and constant regions of an inu-nunoglobulin light chain, heavy chain, or both, have been substituted for the corresponding regions from another immunoglobulin light chain or heavy chain.

The methods of the invention may be practiced on any mammal, preferably on humans.
"amino acid"- a monomeric unit of a peptide, polypeptide, or protein. There are twenty amino acids found in naturally occurring peptides, polypeptides and proteins, all of which are L-isomers. The term also includes analogs of the amino acids and D-isomers of the protein amino acids and their analogs.
"protein"- any polymer consisting essentially of any of the 20 amino acids. Although "polypeptide" is often used in reference to relatively large polypeptides, and "peptide" is often used in reference to small polypeptides, usage of these terms in the art overlaps and is varied. The term "protein" as used herein refers to peptides, proteins and polypeptides, unless otherwise noted.
"fusion"- refers to a co-linear linkage of two or more proteins or fragments thereof via their individual peptide backbones through genetic expression of a polynucleotide molecule encoding those proteins or through protein synthesis methods. It is preferred that the proteins or fragments thereof be from different sources. Thus, preferred fusion proteins include an CD2 binding agent that is a protein or fragment covalently linked to a second moiety that is either a different CD2 binding agent or that is not a CD2 binding agent at all. Specifically, a "CD2 binding agent protein/ Ig fusion" is a protein comprising a CD2 binding agent of the invention, or fragment thereof linked to an N-terminus of an immunoglobulin chain wherein a portion of the N-terminus of the immunoglobulin is replaced with the CD2 binding agent.
"mutant" - any change in the genetic material of an organism, in particular any change (i.e., deletion, substitution, addition, or alteration) in a wild-type polynucleotide sequence or any change in a wild-type protein.
"standard hybridization conditions"- salt and temperature conditions substantially equivalent to 0.5 X SSC to about 5 X SSC and 65 ° C for both hybridization and wash. The term "standard hybridization conditions" as used herein is therefore an operational definition and encompasses a range of hybridization conditions. Higher stringency conditions may, for example, include hybridizing with plaque screen buffer (0.2% polyvinylpyrrolidone, 0.2% Ficoll 400; 0.2% bovine serum albumin, 50 mM Tris-HCl (pH 7.5); 1 M NaCl; 0.1% sodium pyrophosphate; 1 % SDS); 10% dextran sulphate, and 100 µg/ml denatured, sonicated salmon sperm DNA at 65 °C for 12-20 hours, and washing with 75 mM NaCl/7.5 mM sodium citrate (0.5 x SSC)/1% SDS at 65° C. Lower stringency conditions may, for example, include hybridizing with plaque screen buffer, 10% dextran sulphate and 110 µg/ml denatured, sonicated salmon sperm DNA at 55 ° C for 12-20 hours, and washing with 300 mM NaCl/30mM sodium citrate (2.0 X SSC)/1% SDS at 55 ° C. See also **Current Protocols in Molecular Biology,** John Wiley & Sons, Inc. New York, Sections 6.3.1-6.3.6, (1989).
"expression control sequence"- a sequence of polynucleotides that controls and regulates expression of genes when operatively linked to those genes.
"operatively linked"- a polynucleotide sequence (DNA, RNA) is operatively linked to an expression control sequence when the expression control sequence controls and regulates the transcription and translation of that polynucleotide sequence. The term "operatively linked" includes having an appropriate start signal (e.g., ATG) in front of the polynucleotide sequence to be expressed and maintaining the correct reading frame to permit expression of the polynucleotide sequence under the control of the expression control sequence and production of the desired polypeptide encoded by the polynucleotide sequence.
"expression vector"- a polynucleotide, such as a DNA plasmid or phage (among other common examples) which allows expression of at least one gene when the expression vector is introduced into a host cell. The vector may, or may not, be able to replicate in a cell.
"Isolated" (used interchangeably with "substantially pure")- when applied to nucleic acid i.e., polynucleotide sequences, that encode polypeptides, means an RNA or DNA polynucleotide, portion of genomic polynucleotide, cDNA or synthetic polynucleotide which, by virtue of its origin or manipulation: (i) is not associated with all of a polynucleotide with which it is associated in nature (e.g., is present in a host cell as an expression vector, or a portion thereof); or (ii) is linked to a nucleic acid or other chemical moiety other than that to which it is linked in nature; or (iii) does not occur in nature. By "isolated" it is further meant a polynucleotide sequence that is: (i) amplified *in vitro* by, for example, polymerase chain reaction (PCR); (ii) chemically synthesized; (iii) recombinantly produced by cloning; or (iv) purified, as by cleavage and gel separation.

Thus, "substantially pure nucleic acid" is a nucleic acid which is not immediately contiguous with one or both of the coding sequences with which it is normally contiguous in the naturally occurring genome of the organism from which the nucleic acid is derived.

"Isolated" (used interchangeably with "substantially pure")- when applied to polypeptides means a polypeptide or a portion thereof which, by virtue of its origin or manipulation: (i) is present in a host cell as the expression product of a portion of an expression vector; or (ii) is linked to a protein or other chemical moiety other than that to which it is linked in nature; or (iii) does not occur in nature. By "isolated" it is further meant a protein that is : (i) chemically synthesized; or (ii) expressed in a host cell and purified away from associated proteins. The term generally means a polypeptide that has been separated from other proteins and nucleic acids with which it naturally occurs. Preferably, the polypeptide is also separated from substances such as antibodies or gel matrices (polyacrylamide) which are used to purify it.
"heterologous promoter"- as used herein is a promoter which is not naturally associated with a gene or a purified nucleic acid.
"Homologous"- as used herein is synonymous with the term "identity" and refers to the sequence similarity between two polypeptides, molecules or between two nucleic acids. When a position in both of the two compared sequences is occupied by the same base or amino acid monomer subunit (for instance, if a position in each of the two DNA molecules is occupied by adenine, or a position in each of two polypeptides is occupied by a lysine), then the respective molecules are homologous at that position. The percentage homology between two sequences is a function of the number of matching or homologous positions shared by the two sequences divided by the number of positions compared x 100. For instance, if 6 of 10 of the positions in two sequences are matched or are homologous, then the two sequences are 60% homologous. By way of example, the DNA sequences CTGACT and CAGGTT share 50% homology (3 of the 6 total positions are matched). Generally, a comparison is made when two sequences are aligned to give maximum homology. Such alignment can be provided using, for instance, the method of Needleman et al., *J. Mol Biol*. 48: 443-453 (1970), implemented conveniently by computer programs such as the Align program (DNAstar, Inc.). Homologous sequences share identical or similar amino acid residues, where similar residues are conservative substitutions for, or "allowed point mutations" of, corresponding amino acid residues in an aligned reference sequence. In this regard, a "conservative substitution" of a residue in a reference sequence are those substitutions that are physically or functionally similar to the corresponding reference residues, e.g., that have a similar size, shape, electric charge, chemical properties, including the ability to form covalent or hydrogen bonds, or the like. Particularly preferred conservative substitutions are those fulfilling the criteria defined for an "accepted point mutation" in Dayhoff et al., 5: **Atlas of Protein Sequence and Structure,** 5: Suppl. 3, chapter 22: 354-352, Nat. Biomed. Res. Foundation, Washington, D.C. (1978). "Homology" and "identity" each refer to sequence similarity between two polypeptide sequences, with identity being a more strict comparison. Homology and identity can each be determined by comparing a position in each sequence which may be aligned for purposes of comparison. When a position in the compared sequence is occupied by the same amino acid residue, then the polypeptides can be referred to as identical at that position; when the equivalent site is occupied by the same amino acid (e.g., identical) or a similar amino acid (e.g., similar in steric and/or electronic nature), then the molecules can be refered to as homologous at that position. A percentage of homology or identity between sequences is a function of the number of matching or homologous positions shared by the sequences. An "unrelated" or "non-homologous" sequence shares less than 40 percent identity, though preferably less than 25 percent identity, with an AR sequence of the present invention.

Various alignment algorithms and/or programs may be used, including FASTA, BLAST or ENTREZ. FASTA and BLAST are available as a part of the GCG sequence analysis package (University of Wisconsin, Madison, Wis.), and can be used with, e.g., default settings. ENTREZ is available through the National Center for Biotechnology Information, National Library of Medicine, National Institutes of Health, Bethesda, Md. In one embodiment, the percent identity of two sequences can be determined by the GCG program with a gap weight of 1, e.g., each amino acid gap is weighted as if it were a single amino acid or nucleotide mismatch between the two sequences.

The terms "peptide(s)", "protein(s)" and "polypeptide(s)" are used interchangeably herein. The terms "polynucleotide sequence" and "nucleotide sequence" are also used interchangeably herein.

A molecule of the invention is in an "effective amount" if it is that amount which produces a result or exerts an influence on the particular condition being treated.

A "polymer" is a larger molecule constructed from many smaller structural units called "monomers", linked together in any conceivable pattern. When only one species of monomer is used to build a larger molecule, the product is called a "homopolymer", used interchangeably with "polymer". If the chains are composed of more than one different monomer, the material is generically called a "heteropolymer". The polymer moiety to which is attached a CD2 binding agent or fragment or variant is preferably a polyalkylene glycol polymer but any polymer backbone can be used, most preferably those that are water soluble, non-toxic, and non-immunogenic.

Practice of the present invention will employ, unless indicated otherwise, conventional techniques of cell biology, cell culture, molecular biology, microbiology, recombinant DNA, protein chemistry, and immunology, which are within the skill of the art. Such techniques are described in the literature. See, for example, **Molecular Cloning: A Laboratory Manual,** 2nd edition. (Sambrook, Fritsch and Maniatis, eds.), Cold Spring Harbor Laboratory Press, 1989; **DNA Cloning,** Volumes I and II (D.N. Glover, ed), 1985; **Oligonucleotide Synthesis,** (M.J. Gait, ed.), 1984; U.S. Patent No. 4,683,195 (Mullis et al.,); Nucleic Acid Hybridization (B.D. Hames and S.J. Higgins, eds.), 1984; Transcription and Translation (B.D. Hames and S.J. Higgins, eds.), 1984; **Culture of** **Animal Cells** (R.I. Freshney, ed). Alan R. Liss, Inc., 1987; **Immobilized Cells and Enzymes, IRL** Press, 1986; **A Practical Guide to Molecular Cloning** (B. Perbal), 1984; **Methods in Enzymology,** Volumes 154 and 155 (Wu et al., eds), Academic Press, New York; **Gene Transfer Vectors for Mammalian Cells** (J.H. Miller and M.P. Calos, eds.), 1987, Cold Spring Harbor Laboratory; **Immunochemical Methods in Cell and Molecular Biology** (Mayer and Walker, eds.), Academic Press, London, 1987; Handbook of **Experiment Immunology,** Volumes I-IV (D.M. Weir and C.C. Blackwell, eds.), 1986; **Manipulating the Mouse Embryo,** Cold Spring Harbor Laboratory Press, 1986.

While not wishing to be bound by theory, applicants believe that CD2 binding agents used in accordance with the methods of this invention are prophylactic and therapeutic for the treatment of the aforementioned conditions because they either modulate the LFA3/CD2 interaction and/or modulate CD2 signaling, resulting in, among other things, an inhibition of T cell proliferation and activation and, more particularly, a modulation in the number and/or distribution of memory effector T lymphocytes.

Applicants believe that adverse effects of conditions of the type discussed herein are due to such T cell proliferation and activation. Applicants believe that the present invention is superior to previously available therapies for these conditions for a number of reasons, including, inhibition of antigen specific interactions for all antigens present, inhibition of T cell activation, no general immunosuppression and, possibly, induction of tolerance. In particular, applicants believe that use of the agents of this invention will result in more specific targeting of therapy to T cells actually in the initiating stage of the lesion with no effect on polymorphonuclear leukocytes or macrophage mediated effector mechanisms. Accordingly, the patient will be less susceptible to infections than with steroids or other general immunosuppressants. Thus, methods of inhibiting T cell activation, as provided herein, are prophylactic and therapeutic for such skin conditions.

### CD2 Binding Agents

CD2 binding agents useful in the invention have their salient property, the ability to modulate CD2 signaling and to modulate, and preferably inhibit the CD2/LFA-3 interaction. A given CD2 binding agent may have the ability to perform both these functions. Such agents include anti-LFA-3 antibody homologs, soluble LFA-3 polypeptides, soluble CD2 polypeptides, LFA-3 and CD2 mimetic agents and derivatives thereof. Preferred CD2 binding agents are soluble CD2 and LFA-3 polypeptides, and LFA-3 and CD2 mimetic agents.

The utility in this invention of specific soluble CD2 polypeptides, soluble LFA-3 polypeptides, anti-LFA-3 antibody homologs, or CD2 and LFA-3 mimetic agents may be determined, for example, by assaying their ability to modulate the LFA-3/CD2 interaction. This ability may be assayed, for example, using a simple cell binding assay that permits visual (under magnification) evaluation of the ability of the putative CD2 binding agent to inhibit the interaction between LFA-3 and CD2 on cells bearing these molecules. Jurkat cells are preferred as the CD2+ substrate and sheep red blood cells or human JY cells are preferred as the LFA-3+ substrate. The binding characteristics of soluble polypeptides, antibody homologs and mimetic agents useful in this invention may be assayed in several known ways, such as by radiolabeling the antibody homolog, polypeptide or agent (e.g., 35S or 125I) and then contacting the labeled polypeptide, mimetic agent or antibody homolog with CD2+ of LFA-3+ cells, as appropriate. Binding characteristics may also be assayed using an appropriate enzymatically labelled secondary antibody. Rosetting competition assays such as those described by Seed et al. (Proc, Natl. Acad, Sci, U A, 84, pp. 3365-69 (1987)) may also be used.

### A. Anti-LFA-3 Homologs

Many types of anti-LFA-3 homologs are useful in this invention. These include monoclonal antibodies, recombinant antibodies, chimeric recombinant antibodies, humanized recombinant antibodies, as well as antigen-binding portions of the foregoing.

Among the anti-LFA-3 antibody homologs, it is preferable to use monoclonal anti-LFA-3 antibodies. It is more preferable to use a monoclonal anti-LFA-3 antibody produced by a hybridoma selected from the group of hybridomas having Accession Nos. ATCC HB 10693 (1E6), ATCC HB 10694 (HC- 1B11), ATCC HB 10695 (7A6), and ATCC HB 10696 (8B8), or the monoclonal antibody known as TS2/9 (Sanchez-Madrid et al., "Three Distinct Antigens Associated with Human T-Lymphocyte-Mediated Cytolysis: LFA-1, LFA-2 and LFA-3", Proc, Natl, Acad. Sci, USA, 79, pp. 7489-93 (1982)). Most preferably, the monoclonal anti-LFA-3 antibody is produced by a hybridoma selected from the group of hybridomas having Accession Nos. ATCC HB 10695 (7A6) and ATCC HB 10693 (I E6).

The technology for producing monoclonal antibodies is well known. Briefly, an immortal cell line (typically myeloma cells) is fused to lymphocytes (typically splenocytes) from a mammal immunized with preparation comprising a given antigen, and the culture supernatants of the resulting hybridoma cells are screened for antibodies against the antigen. See generally, Kohler et al., Nature, "Continuous Cultures of Fused Cells Secreting Antibody of Predefined Specificity", 256, pp. 495-97 (1975). Useful immunogens for the purpose of this invention include LFA-3-bearing cells, as well as cell free preparations containing LFA-3, or counter receptor-binding fragments thereof (e.g., CD2 fragments that bind to LFA-3 or LFA-3 fragments that bind to CD2).

Immunization may be accomplished using standard procedures. The unit dose and immunization regimen depend on the species of mammal immunized, its immune status, the body weight of the mammal, etc. Typically, the immunized mammals are bled and the serum from each blood sample is assayed for particular antibodies using appropriate screening assays. For example, useful anti-LFA-3 antibodies may be identified by testing the ability of the immune serum to block sheep red blood cell resetting of Jurkat cells, which results from the presence of LFA-3 on the respective surfaces of these cells. The lymphocytes used in the production of hybridoma cells typically are isolated from immunized mammals whose sera have already tested positive for the presence of the desired antibodies using such screening assays.

Typically, the immortal cell line (e.g., a myeloma cell line) is derived from the same mammalian species as the lymphocytes. Preferred immortal cell lines are mouse myeloma cell lines that are sensitive to culture medium containing hypoxanthine, aminopterin and thymidine ("HAT medium"). Typically, HAT-sensitive mouse myeloma cells are fused to mouse splenocytes using polyethylene glycol ("PEG") 3350. Hybridoma cells resulting from the fusion are then selected using HAT medium, which kills unfused and unproductively fused myeloma cells (unfiised splenocytes die after several days because they are not transformed). Hybridomas producing a desired antibody are detected by screening the hybridoma culture supernatants, for example, for the ability to bind to their respective counter receptor, or for their ability to block Jurkat cell adhesion to sheep red blood cells. Subcloning of the hybridoma cultures by limiting dilution is typically performed to ensure monoclonality.

To produce anti-LFA-3 monoclonal antibodies, hybridoma cells that tested positive in such screening assays are cultured in a nutrient medium under conditions and for a time sufficient to allow the hybridoma cells to secrete the monoclonal antibodies into the culture medium. Tissue culture techniques and culture media suitable for hybridoma cells are well known. The conditioned hybridoma culture supernatant may be collected and the desired antibodies optionally further purified by well-known methods.

Alternatively, the desired antibody may be produced by injecting the hybridoma cells into the peritoneal cavity of a pristane-primed mouse. The hybridoma cells proliferate in the peritoneal cavity, secreting the antibody, which accumulates as ascites fluid. The antibody may be harvested by withdrawing the ascites fluid from the peritoneal cavity with a syringe.

Anti-LFA-3 antibody homologs useful in the present invention may also be recombinant antibodies produced by host cells transformed with DNA encoding immunoglobulin light and heavy chains of a desired antibody. Recombinant antibodies may be produced by well known genetic engineering techniques. See, e.g., U.S. Patent No. 4,816,397. For example, recombinant antibodies may be produced by cloning cDNA or genomic DNA encoding the immunoglobulin light and heavy chains of the desired antibody from a hybridoma cell that produces an antibody homolog useful in this invention. The cDNA or genomic DNA encoding those polypeptides is then inserted into expression vectors so that both genes are operatively linked to their own transcriptional and translational expression control sequences. The expression vector and expression control sequences are chosen to be compatible with the expression host cell used. Typically, both genes are inserted into the same expression vector. Prokaryotic or eukaryotic host cells may be used. Expression in eukaryotic host cells is preferred because such cells are more likely than prokaryotic cells to assemble and secrete a properly folded and immunologically active antibody. However, any antibody produced that is inactive due to improper folding may be renaturable according to well known methods (Kim and Baldwin, "Specific Intermediates in the Folding Reactions of Small Proteins and the Mechanism of Protein Folding", Ann, Rev. Biochem., 5 1, pp. 459-89 (1982)). It is possible that the host cells will produce portions of intact antibodies, such as light chain dimers or heavy chain dimers, which also are antibody homologs according to the present invention.

It will be understood that variations on the above procedure are useful in the present invention. For example, it may be desired to transform a host cell with DNA encoding either the light chain or the heavy chain (but not both) of an antibody homolog. Recombinant DNA technology may also be used to remove some or all of the DNA encoding either or both of the light and heavy chains that is not necessary for CD2 or LFA-3 counter receptor binding. The molecules expressed from such truncated DNA molecules are useful in the methods of this invention. In addition, bifunctional antibodies may be produced in which one heavy and one light chain are anti-LFA-3 antibody homologs and the other heavy and light chain are specific for an antigen other than LFA-3, or another epitope of LFA-3.

Chimeric recombinant anti-LFA-3 antibody homologs may be produced by transforming a host cell with a suitable expression vector comprising DNA encoding the desired immunoglobulin light and heavy chains in which all or some of the DNA encoding the hinge and constant regions of the heavy and/or the light chain have been substituted with DNA from the corresponding region of an immunoglobulin light or heavy chain of a different species. When the original recombinant antibody is nonhuman, and the CD2 binding agent is to be administered to a human, substitution of corresponding human sequences is preferred. An exemplary chimeric recombinant antibody has mouse variable regions and human hinge and constant regions. See generally, U.S. Patent No. 4,816,397 and Morrison et al., "Chimeric Human Antibody Molecules: Mouse Antigen-Binding Domains With Human Constant Region Domains", Proc, Natl. Acad. Sci, USA, 8 1, pp. 6851-55 (1984). Humanized recombinant anti-LFA-3 antibodies may be produced by transforming a host cell with a suitable expression vector comprising DNA encoding the desired nonhuman immunoglobulin light and heavy chains in which all or some of the DNA encoding amino acids not involved in antigen binding have been substituted with DNA from the corresponding region of a desired human immunoglobulin light or heavy chain. See generally, Jones et al., "Replacing the Complementarity-Determining Regions in a Human Antibody with Those from a Mouse", Nature, 321, pp. 522-25 (1986).

Anti-LFA-3 antibody homologs that are not intact antibodies are also useful in this invention. Such homologs may be derived from any of the antibody homologs described above. For example, antigen-binding fragments, as well as full-length monomeric, dimeric or trimeric polypeptides derived from the above-described antibodies are themselves useful.. Useful antibody homologs of this type include Fab fragments, Fab'fragments, F(ab')2 fragments, F(v) fragments, heavy chain monomers or dimers, light chain monomers or dimers, dimers consisting of one heavy and one light chain, and the like. Anti-LFA-3 heavy chains are preferred anti-LFA-3 antibody fragments.

Antibody fragments may also be produced by chemical methods, e.g., by cleaving an intact antibody with a protease, such as pepsin or papain, and optionally treating the cleaved product with a reducing agent. Alternatively, useful fragments may be produced by using host cells transformed with truncated heavy and/or light chain genes. Heavy and light chain monomers may be produced by treating an intact antibody with a reducing agent, such as dithiothreitol, followed by purification to separate the chains. Heavy and light chain monomers may also be produced by host cells transformed with DNA encoding either the desired heavy chain or light chain, but not both. See, e.g., Ward et al., "Binding Activities of a Repertoire of Single Immunoglobulin Variable Domains Secreted from Escherichia coli", Nature, 341, pp. 544-46 (1989); Sastry et al., "Cloning of the Immunological Repertoire in Escherichia coli for Generation of Monoclonal Catalytic Antibodies: Construction of a Heavy Chain Variable Region-Specific cDNA Library", Proc. Natl. Acad. Sci, USA, 86, pp. 572832 (1989).

### B. Soluble CD2 and LFA-3 Polypeptides

Soluble LFA-3 polypeptides or soluble CD2 polypeptides that modulate the interaction of LFA-3 and CD2 are useful in the methods of the present invention. Soluble LFA-3 polypeptides are preferred.

Soluble LFA-3 polypeptides may be derived from the transmembrane form of LFA-3, particularly the extracellular domain (e.g.,AA 1-AA187 of SEQ ID NO:2). Such polypeptides are described in U.S. Patent No. 4,956,281 and U.S. Patent No. 5,547,853 (which shares a common assignee with the present application).

Preferred soluble LFA-3 polypeptides include polypeptides consisting of AA 1-AA 92 of SEQ ID NO:2, AA 1-AA 80 of SEQ ID NO:2, AA 50-AA 65 of SEQ ID NO:2 and AA 20-AA 80 of SEQ ID NO:2. A vector comprising a DNA sequence (SEQ ID NO: 1) that encodes the amino acids of SEQ ID NO:2 has been deposited with the American Type Culture Collection, Rockville, Maryland under Accession No. 75107.

The most preferred proteins of this type are fusion proteins that contain the amino terminal 92 amino acids of mature LFA-3, the C-terminal 10 amino acids of a human IgG 1 hinge region containing the two cysteine residues thought to participate in interchain disulfide bonding, and the CH2 and CH3 regions of a human IgGI heavy chain constant domain (e.g., SEQ ID NO: 8). This fusion protein is referred to herein as "LFA3TIP." A plasmid, pSAB 152, encoding an exemplary LFA3TIP has been deposited with the American Type Culture Collection, Rockville, Maryland, under the accession number ATCC 68720. The DNA sequence of the pSAB152 insert is SEQ ID NO:7. This recombinant protein has been designed to modulate immune responses through interaction with the CD2 receptor. The LFA-3 portion of the fusion binds to the CD2 receptor on T lymphocytes. The IgG₁ portion binds to the FcgammaRI (macrophage) and FcgammaRIII (NK cells and neutrophils) receptors. The interaction of LFA3TIP with CD2 has been shown to inhibit in vitro human T lymphocyte responses. See United States Patent 5,728,677 and 5,547,853. One way of producing LFA3TIP for use in the methods of this invention is described in United States Patent 5,547,853.

Generally, conditioned culture medium of COS7 or CHO cells transfected with pSAB 152 is concentrated using an AMICON SI Y3 0 spiral cartridge system (AMICON, Danvers, Massachusetts) and subjected to Protein A-Sepharose 4B (Sigma, St. Louis, Missouri) chromatography. The bound proteins are eluted and subjected to Superose-12 (Pharmacia/LKB, Piscataway, New Jersey) gel filtration chromatography. Superose-12 fractions containing LFA3TIP with the least amount of contaminating proteins, as determined on SDS-PAGE gels and by Western blot analysis, (see, e.g., Towbin et al., Proc, Natl. Acad, Sci, USA, 74, pp. 4350-54 (1979); Antibodies: A Laboratory Manual, pp. 474-5 10 (Cold Spring Harbor Laboratory (I 98 8)), are pooled and concentrated in a YM30 Centricon (AMICON). LFA3TIP is detected on Western blots using a rabbit anti-LFA-3 polyclonal antiserum, followed by delectably labeled goat anti-rabbit IgG. The purified LFA3TIP of COS7 or CHO cells is a dimer of two monomeric LFA-3-Ig fusion proteins, connected by disulfide bonds.

Another preferred fusion protein consists of the first and second LFA-3 extracellular domains fused to the hinge CH2 and CH3 regions of human IgGl, herein referred to as LLFA3-Ig.

Soluble LFA-3 polypeptides may also be derived from the PI-linked form of LFA-3, such as those described in WO 90/0218 1. A vector comprising a DNA sequence encoding PI-linked LFA-3 (i.e., SEQ ID NO:3) is deposited with the American Type Culture Collection, Rockville, Maryland under Accession No. 68788. It is to be understood that the PI-linked form of LFA-3 and the transmembrane form of LFA-3 have identical amino acid sequences through the entire extracellular domain.

Accordingly, the preferred PI-linked LFA-3 polypeptides are the same as for the transmembrane form of LFA-3. Soluble CD2 polypeptides may be derived from full length CD2, particularly the extracellulardomain (e.g.,AA 1-AA 185 of SEQ ID NO:6). Such polypeptides may comprise all or part of the extracellular domain of CD2. Exemplary soluble CD2 polypeptides are described in WO 90/08187, which is herein incorporated by reference.

The production of the soluble polypeptides useful in this invention may be achieved by a variety of methods known in the art. For example, the polypeptides may be derived from intact transmembrane LFA-3 or CD2 molecules or an intact PI-linked LFA-3 molecule by proteolysis using specific endopeptidases in combination with exopeptidases, Edman degradation, or both. The intact LFA-3 molecule or the intact CD2 molecule, in turn, may be purified from its natural source using conventional methods. Alternatively, the intact LFA-3 or CD2 may be produced by known recombinant DNA techniques using cDNAs (see, e.g., U.S. Patent No. 4,956,281 to Wallner et al.; Aruffo and Seed, Proc. Natl. Acad. Sci., 84, pp. 2941-45 (1987); Sayre et al., Proc, Natl. Acad, Sci. USA, 84, pp. 2941-45 (1987)).

Preferably, the soluble polypeptides useful in the present invention are produced directly, thus eliminating the need for an entire LFA-3 molecule or an entire CD2 molecule as a starting material. This may be achieved by conventional chemical synthesis techniques or by well-known recombinant DNA techniques wherein only those DNA sequences which encode the desired peptides are expressed in transformed hosts. For example, a gene which encodes the desired soluble LFA-3 polypeptide or soluble CD2 polypeptide may be synthesized by chemical means using an oligonucleotide synthesizer. Such oligonucleotides are designed based on the amino acid sequence of the desired soluble LFA-3 polypeptide or soluble CD2 polypeptide. Specific DNA sequences coding for the desired peptide also can be derived from the full length DNA sequence by isolation of specific restriction endonuclease fragments or by PCR synthesis of the specified region.

Standard methods may be applied to synthesize a gene encoding a soluble LFA-3 polypeptide or a soluble CD2 polypeptide that is useful in this invention. For example, the complete amino acid sequence may be used to construct a back-translated gene. A DNA oligomer containing a nucleotide sequence coding for a soluble LFA-3 polypeptide or a soluble CD2 polypeptide useful in this invention may be synthesized in a single step. Alternatively, several smaller oligonucleotides coding for portions of the desired polypeptide may be synthesized and then ligated. Preferably, a soluble LFA-3 polypeptide or a soluble CD2 polypeptide useful in this invention will be synthesized as several separate oligonucleotides which are subsequently linked together. The individual oligonucleotides typically contain 5' or 3' overhangs for complementary assembly. Once assembled, preferred genes will be characterized by sequences that are recognized by restriction endonucleases (including unique restriction sites for direct assembly into a cloning or an expression vector), preferred codons taking into consideration the host expression system to be used, and a sequence which, when transcribed, produces a stable, efficiently translated MRNA. Proper assembly may be confirmed by nucleotide sequencing, restriction mapping, and expression of a biologically active polypeptide in a suitable host.

It will be appreciated by those of skill in the art that, due to the degeneracy of the genetic code, DNA molecules comprising many other nucleotide sequences will also be capable of encoding the soluble LFA-3 and CD2 polypeptides encoded by the specific DNA sequences described above. These degenerate sequences also code for polypeptides that are useful in this invention.

The DNA sequences may be expressed in unicellular hosts. As is well known in the art, in order to obtain high expression levels of a transfected gene in a host, the gene must be operatively linked to transcriptional and translational expression control sequences that are functional in the chosen expression host. Preferably, the expression control sequences, and the gene of interest, will be contained in an expression vector that further comprises a bacterial selection marker and origin of replication. If the expression host is a eukaryotic cell, the expression vector should further comprise an additional expression marker useful in the expression host. The DNA sequences encoding the desired soluble polypeptides may or may not encode a signal sequence. If the expression host is prokaryotic, it generally is preferred that the DNA sequence not encode a signal sequence. If the expression host is eukaryotic, it generally is preferred that a signal sequence be encoded. An amino terminal methionine may or may not be present on the expressed product. If the terminal methionine is not cleaved by the expression host, it may, if desired, be chemically removed by standard techniques.

A wide variety of expression host/vector combinations may be employed. Useful expression vectors for eukaryotic hosts, include, for example, vectors comprising expression control sequences from SV40, bovine papilloma virus, adenovirus and cytomegalovirus. Useftil expression vectors for bacterial hosts include known bacterial plasmids, such as plasmids from E. coli, including col El, PCRI, pBR322, pMB9 and their derivatives, wider host range plasmids, such as RP4, phage DNAs, e.g., the numerous derivatives of phage lambda, e. g., NM989, and other DNA phages, such as M13 and filamentous single stranded DNA phages. Useful expression vectors for yeast cells include the 2µ plasmid and derivatives thereof. Useful vectors for insect cells include pVL 941. In addition, any of a wide variety of expression control sequences may be used in these vectors. Such useful expression control sequences include the expression control sequences associated with structural genes of the foregoing expression vectors. Examples of useful expression control sequences include, for example, the early and late promoters of SV40 or adenovirus, the lac system, the trp system, the TAC or TRC system, the major operator and promoter regions of phage lambda, the control regions of fd coat protein, the promoter for 3-phosphoglycerate kinase or other glycolytic enzymes, the promoters of acid phosphatase, e.g., Pho5, the promoters of the yeast a-mating system and other sequences known to control the expression of genes of prokaryotic or eukaryotic cells or their viruses, and various combinations thereof.

A wide variety of unicellular host cells are useful. These hosts may include well known eukaryotic and prokaryotic hosts, such as strains of E. coli, Pseudomonas, Bacillus, Streptomyces, fungi, yeast, insect cells such as Spodoptera frugiperda (SF9), animal cells such as CHO and mouse cells, African green monkey cells such as COS 1, COS 7, BSC 1, BSC 40, and BMT 10, and human cells, as well as plant cells in tissue culture. For animal cell expression, we prefer C140 cells and COS 7 cells. It should, of course, be understood that not all vectors and expression control sequences will function equally well to express the DNA sequences described herein. Neither will all hosts function equally well with the same expression system. However, one of skill in the art may make a selection among these vectors, expression control sequences and hosts without undue experimentation. For example, in selecting a vector, the host must be considered because the vector must replicate in it. The vector's copy number, the ability to control that copy number, and the expression of any other proteins encoded by the vector, such as antibiotic markers, should also be considered.

In selecting an expression control sequence, a variety of factors should also be considered. These include, for example, the relative strength of the sequence, its controllability, and its compatibility with the DNA sequences discussed herein, particularly as regards potential secondary structures. Unicellular hosts should be selected by consideration of their compatibility with the chosen vector, the toxicity of the product coded for by the DNA sequences, their secretion characteristics, their ability to fold the soluble polypeptides correctly, their fermentation or culture requirements, and the ease of purification of the products coded for by the DNA sequences. Within these parameters, one of skill in the art may select various vector/expression control sequence/host combinations that will express the desired DNA sequences on fermentation or in large scale aniinal culture, for example with CHO cells or COS 7 cells.

The soluble LFA-3 and CD2 polypeptides may be isolated from the fermentation or cell culture and purified using any of a variety of conventional methods. One of skill in the art may select the most appropriate isolation and purification techniques.

While recombinant DNA techniques are the preferred method of producing useful soluble CD2 polypeptides or soluble LFA-3 polypeptides having a sequence of more than 20 amino acids, shorter CD2 or LFA-3 polypeptides having less than about 20 amino acids are preferably produced by conventional chemical synthesis techniques. Synthetically produced polypeptides useful in this invention can advantageously be produced in extremely high yields and can be easily purified. Preferably, such soluble CD2 polypeptides or soluble LFA-3 polypeptides are synthesized by solution phase or solid phase polypeptide synthesis and, optionally, digested with carboxypeptidase (to remove C-terminal amino acids) or degraded by manual Edman degradation (to remove N-terminal amino acids). Proper folding of the polypeptides may be achieved under oxidative conditions which favor disulfide bridge formation as described by Kent, "Chemical Synthesis of Polypeptides and Proteins", Ann, Rev. Biochem., 57, pp. 95789 (1988). Polypeptides produced in this way may then be purified by separation techniques widely known in the art, preferably utilizing reverse phase HPLC. The use of solution phase synthesis advantageously allows for the direct addition of certain derivatized amino acids to the growing polypeptide chain, such as the 0-sulfate ester of tyrosine. This obviates the need for a subsequent derivatization step to modify any residue of the polypeptides useful in this invention.

### C. LFA-3 And CD2 Mimetic Agents

Also useful in the methods of this invention are LFA-3 and CD2 mimetic agents. These agents which may be peptides, semi-peptidic compounds or non-peptidic compounds, are CD2 biding agents that modulate CD2 signaling and/or the CD2/LFA-3 interaction. The most preferred CD2 and LFA-3 mimetic agents will inhibit the CD2/LFA-3 interaction at least as well as anti-LFA-3 monoclonal antibody 7A6 or anti-CD2 monoclonal antibody TS2/18 (described supra). Such mimetic agents may be produced by synthesizing a plurality of peptides (e.g., 520 amino acids in length), semi-peptidic compounds or non-peptidic, organic compounds, and then screening those compounds for their ability to inhibit the CD2/LFA-3 interaction. See generally U.S. Patent No. 4,833,092, Scott and Smith, "Searching for Peptide Ligands with an Epitope Library", Science, 249, pp. 386-90 (1990), and Devlin et al., "Random Peptide Libraries: A Source of Specific Protein Binding Molecules", Science, 249, pp. 40407 (1990).

### D. Derivatized CD2 binding agents

Also useful in the methods of this invention are derivatized CD2 binding agents such as modulators of the CD2/LFA-3 interaction in which, for example, any of the antibody homologs, soluble CD2 and LFA-3 polypeptides, or CD2 and LFA-3 mimetic agents described herein are functionally linked (by chemical coupling, genetic fusion or otherwise) to one or more members independently selected from the group consisting of anti-LFA-3 and anti-CD2 antibody homologs, soluble LFA-3 and CD2 polypeptides, CD2 and LFA-3 mimetic agents, cytotoxic agents and pharmaceutical agents. One type of derivatized CD2 binding agent is produced by crosslinking two or more CD2 binding agents (of the same type or of different types). Suitable crosslinkers include those that are heterobifimctional, having two distinctly reactive groups separated by an appropriate spacer (e.g., m-maleimidobenzoyl-N-hydroxysuccinimide ester) or homobiftmctional (e.g., disuccinimidyl suberate). Such linkers are available from Pierce Chemical Company, Rockford, Illinois. Another possibility for cross-linking takes advantage of the PI linkage signal sequence in PI-linked LFA-3, or fragments thereof. Specifically, DNA encoding the Pllinkage signal sequence (e.g., AA162-AA212 of SEQ ID NO:4) is ligated downstream of DNA encoding a desired polypeptide, preferably a soluble LFA-3 polypeptide. If this construct is expressed in an appropriate eukaryotic cell, the cell will recognize the PI linkage signal sequence and will covalently link PI to the polypeptide. The hydrophobic property of the PI may then be exploited to form micellar aggregates of the polypeptides.

Also useful are CD2 binding agents linked to one or more cytotoxic or pharmaceutical agents. Useful pharmaceutical agents include biologically active peptides, polypeptides and proteins, such as antibody homologs specific for a human polypeptide other than CD2 or LFA-3, or portions thereof Useful pharmaceutical agents and cytotoxic agents also include cyclosporin A, prednisone, FK506, methotrexate, steroids, retinoids, interferon, and nitrogen mustard.

Preferred CD2 binding agents derivatized with a pharmaceutical agent include recombinantly produced polypeptides in which a soluble LFA-3 polypeptide, soluble CD2 polypeptide, or a peptidyl CD2 or peptidyl LFA-3 mimetic agent is fused to all or part of an immunoglobulin heavy chain hinge region and all or part of a heavy chain constant region. Preferred polypeptides for preparing such fusion proteins are soluble LFA-3 polypeptides. Most preferred are fusion proteins containing AA 1-AA 92 of LFA-3 (e.g., SEQ ID NO:2) fused to a portion of a human IgG I hinge region (including the C-terminal ten amino acids of the hinge region containing two cysteine residues thought to participate in interchain disulfide bonding) and the CH2 and CH3 regions of an IgG1 heavy chain constant domain. Such fusion proteins are expected to exhibit prolonged serum half-lives and enable CD2 binding agent dimerization.

Other derivatized CD2 binding agents also expected to exhibit prolonged serum half lives are CD2 binding agents linked most preferably to one or more polymers comprising a polyalkylene glycol polymer. The polymer would be expected to selectively react with free amino or other reactive groups on a CD2 binding agent and in theory, the polymer(s) are reacted so that attachment could occur at any available amino group such as alpha amino groups or the epsilon-amino groups of lysines, or -SH groups of cysteines. Free carboxylic groups, suitably activated carbonyl groups, hydroxyl, guanidyl, oxidized carbohydrate moieties and mercapto groups of the CD2 binding agent (if available) can also be used as attachment sites.

In particular, if the CD2 binding agent is a protein, the chemical modification of any cysteine (e.g., an internal or an N-terminal cysteine) to protect the thiol, with concomitant conjugation with a polyalkylene glycol moiety (i.e., polyethylene glycol or PEG), can be carried out in numerous ways by someone skilled in the art. See United States Patent 4,179,337. The sulfhydryl moiety, with the thiolate ion as the active species, is the most reactive functional group in a protein. There are many reagents that react faster with the thiol than any other groups. See Chemistry of Protein Conjugation and Cross-Linking (S. S. Wong, CRC Press, Boca Raton, FL, 1991).

Other examples of methods that provide linkage between a polyalkylene glycol and a cysteine would be reactions with other alpha-haloacetyl compounds, organomercurials, disulfide reagents, and other N-substituted maleimides. Numerous derivatives of these active species are available commercially (e.g., ethyl iodoacetate (Aldrich, Milwaukee WI), phenyl disulfide (Aldrich), and N-pyrenemaleimide (Molecular Probes, Eugene OR)) or could be synthesized readily (e.g., N-alkyliodoacetamides, N-alkylmaleimides, and organomercurials). While there is no simple chemical strategy for targeting a polyalkylene glycol polymer such as PEG to a CD2 binding agent, it is straightforward to genetically engineer a site that can be used to target the polymer moiety, such as using site-directed mutagenesis. For example, incorporation of a Cys at a site that is at or near the C-terminus of a proteinaceous CD2 binding agent allows specific modification using a maleimide, vinylsulfone or haloacetate- activated polyalkylene glycol (e.g., PEG). The reactions may take place by any suitable method used for reacting biologically active materials with inert polymers.

Generally the process involves preparing an activated polymer (that may have at least one terminal hydroxyl group) and thereafter reacting the protein with the activated polymer to produce the soluble protein suitable for formulation. The above modification reaction can be performed by several methods, which may involve one or more steps. In the practice of the present invention, polyalkylene glycol residues of C1-C4 alkyl polyalkylene glycols, preferably polyethylene glycol (PEG), or poly(oxy)alkylene glycol residues of such glycols are advantageously incorporated in the polymer systems of interest. Thus, the polymer to which the protein is attached can be a homopolymer of polyethylene glycol (PEG) or is a polyoxyethylated polyol, provided in all cases that the polymer is soluble in water at room temperature. Non-limiting examples of such polymers include polyalkylene oxide homopolymers such as PEG or polypropylene glycols, polyoxyethylenated glycols, copolymers thereof and block copolymers thereof, provided that the water solubility of the block copolymer is maintained.

Examples of polyoxyethylated polyols include, for example, polyoxyethylated glycerol, polyoxyethylated sorbitol, polyoxyethylated glucose, or the like. The glycerol backbone of polyoxyethylated glycerol is the same backbone occurring naturally in, for example, animals and humans in mono-, di-, and triglycerides. Therefore, this branching would not necessarily be seen as a foreign agent in the body.

As an alternative to polyalkylene oxides, dextran, polyvinyl pyrrolidones, polyacrylamides, polyvinyl alcohols, carbohydrate-based polymers and the like may be used. Moreover, heteropolymers (i.e., polymers consisting of more than one species of monomer such as a copolymer) as described in U.S. Patent 5,359,030 may be used (e.g., proteins conjugated to polymers comprising a polyalkylene glycol moiety and one or more fatty acids) Those of ordinary skill in the art will recognize that the foregoing list is merely illustrative and that all polymer materials having the qualities described herein are contemplated.

Moreover, in another aspect of the invention, one can utilize a derivatized CD2 binding agent covalently bonded to the polymer component in which the nature of the conjugation involves cleavable covalent chemical bonds. This allows for control in terms of the time course over which the polymer may be cleaved from the CD2 binding agent. This covalent bond may be cleaved by chemical or enzymatic reaction. The polymer-CD2 binding agent product retains an acceptable amount of activity. Concurrently, portions of polyethylene glycol are present in the conjugating polymer to endow the polymer-CD2 binding agent conjugate with high aqueous solubility and prolonged blood circulation capability. As a result of these improved characteristics the invention contemplates parenteral, aerosol, and oral delivery of both the active polymer-CD2 binding agent species and, following hydrolytic cleavage, bioavailability of the CD2 binding agent per se, in in vivo applications.

### Pharmaceutical Compositions And Uses According To This Invention

This invention provides the use of agents for preventing or treating a condition in a mammal characterized by: the infiltration into one or more organs of the mammal of memory effector T lymphocytes; and/or the association with such condition by such T lymphocytes. The use includes one or more CD2 binding agents such as inhibitors of the CD2/LFA-3 interaction, or derivatized form(s) thereof that are capable of selectively modulating memory effector T lymphocytes as compared to naïve T lymphocytes. Preferably, an effective amount of the CD2 binding agent or derivatized form thereof is to be administered. By "effective amount" is meant an amount capable of lessening the spread or severity of the conditions described herein.

It will be apparent to those of skill in the art that the effective amount of CD2 binding agent will depend, inter alia, upon the administration schedule, the unit dose administered, whether the CD2 binding agent is administered in combination with other therapeutic agents, the immune status and health of the patient, the therapeutic activity of the particular CD2 binding agent administered and the serum half-life.

The CD2 binding agent, or pharmaceutical composition, may be in a variety of forms. These include, for example, solid, semi-solid and liquid dosage forms, such as tablets, pills, powders, liquid solutions, dispersions or suspensions, liposomes, suppositories, injectable infusible, and topical preparations. The preferred form depends on the intended mode of administration and therapeutic application. The preferred forms are injectable or infusible solutions. The CD2 binding agent or pharmaceutical composition may be administered intravenously, intramuscularly, subcutaneously, intra-articularly, intrathecally, periostally, intratumorally, intralesionally, perilesionally by infusion, orally, topically or by inhalation. Preferably it is administered subcutaneously, intramuscularly or intravenously. Most preferably, it is administered subcutaneously. The CD2 binding agents may be administered orally, buccally, parenterally, rectally, vaginally, by intranasal inhalation spray, by intrapulmonary inhalation or in other ways. In particular, the agents according to the invention may be formulated for inhalation with spray or powder, for injection (for example subcutaneous, intramuscular, intravenous, intra-articular or intracisternal injection), for infusion or for oral administration and may be presented in unit dose form in ampoules or tablets or in multi-dose vials or other containers with an added preservative. The compositions may take such forms as suspensions, solutions, or emulsions or gels in oily or aqueous vehicles, and may contain formulatory agents such as suspending, stabilising and/or dispersing agents. Alternatively, the active ingredient may be in powder and/or lyophilised form for direct administration or for constitution with a suitable vehicle (e.g. sterile, pyrogen-free water, normal saline or 5% dextrose) before use.

Preferably, the unit dose is administered about one to three times per week or one to three times per day. More preferably, it is administered about one to three times per day for between about 3 and 7 days, or about one to three times per day for between about 3 and 7 days on a monthly basis. It will be recognized, however, that lower or higher dosages and other administrations schedules may be employed. Preferably, the CD2 binding agent is administered at a dose between about 0.001 and about 50 mg CD2 binding agent per kg body weight, more preferably, between about 0.01 and about 10 mg CD2 binding agent per kg body weight, most preferably between about 0.1 and about 4 mg CD2 binding agent per kg body weight.

For example, to administer a CD2 binding agent of the invention (the soluble LFA-3 polypeptide LFA3TIP) to a patient, a particularly preferred CD2 binding agent is packaged as a frozen solution in vials containing 10 mg/mL recombinant LFA-3/IgG₁ fusion protein and excipient materials (sodium chloride, monobasic potassium phosphate, and dibasic sodium phosphate). The vials are thawed in the refrigerator or at room temperature and diluted with 0.9% sodium chloride to a final volume of 5 mL for IV bolus administration. LFA3TIP, is constructed as described in Miller et al. (1993) *J Exp. Med. 1* 78:211, and is purified from culture medium of transfectant CHO (Chinese Hamster Ovary) cell lines by absorption to Protein-A Sepharose 4B (Pharmacia) and eluted with 50 mM glycine, 250 mM NaCl (pH 3.0). Fractions containing protein are pooled and subjected to gel filtration on Superose-6® (Pharmacia) in phosphate buffered saline (PBS). Peak fractions are pooled and analyzed for purity on 12% reducing and non-reducing SDS-PAGE.

In one exemplary protocol, the patient receive one IV bolus every week at a dosage that preferably ranges greater than 0.025 mg/kg and can be up to between 0.075 mg/kg, through 0.150 mg/kg. Dosing of this CD2 binding agent is based on the subject's baseline body weight. Other dosage regimens can include one intramuscular (IM) injection at a dosage of no less than 0.005 mg/kg, with preferred dosages ranging upwards of 0.025 mg/kg, the most preferred dosages being selected from 0.05, 0.075, 0.1125, and 0.165 mg/kg.

Unit doses should be administered until an effect is observed. The effect may be measured by a variety of methods. In the case of MS, the therapeutic effect can be measured by standard magnetic resonance methods. In the case of IBD, assaying extent of abdominal pain, healing of fistulas, frequency of stools, and the like, in the case of psoriatic arthritis, by changes in joint swelling and range in motion. Persons having ordinary skill in the medical arts will readily be able to measure the clinical effect on any one of the indications described herein by reference to well known medical procedures.

The CD2 binding agent(s) or derivatized form(s) thereof are preferably administered in a composition including a pharmaceutically acceptable carrier. By "pharmaceutically acceptable carrier" is meant a carrier that does not cause an allergic reaction or other untoward effect in patients to whom it is administered. Suitable pharmaceutically acceptable carriers include, for example, one or more of water, saline, phosphate buffered saline, dextrose, glycerol, ethanol and the like, as well as combinations thereof. Pharmaceutically acceptable carriers may further comprise minor amounts of auxiliary substances such as wetting or emulsifying agents, preservatives or buffers, which enhance the shelf life or effectiveness of the CD2 binding agent.

The pharmaceutical composition or CD2 binding agent may be administered in conjunction with other therapeutic or prophylactic agents. These include, for example, cyclosporin A, steroids, retinoids, nitrogen mustard, interferon, methotrexate, antibiotics and antihistamines. These agents may be administered in single dosage form with the CD2 binding agent (i.e., as part of the same pharmaceutical composition), a multiple dosage form separately from the CD2 binding agent, but concurrently, or a multiple dosage form wherein the two components are administered separately but sequentially. Alternatively, the CD2 binding agent and the other active agent may be in the form of a single conjugated molecule. Conjugation of the two components may be achieved by standard cross-linking techniques well known in the art. A single molecule may also take the form of a recombinant fusion protein. In addition, the CD2 binding agents, or pharmaceutical compositions, useful in the present invention may be used in combination with other therapies such as chemotherapy. Such combination therapies may advantageously utilize lower dosages of the therapeutic agents.

### Gene Therapy

The invention is based further on gene therapy based delivery of the CD2 binding agents as expression products of a nucleic acid sequence within a mammalian host. Preferred ex vivo systemic delivery of the gene products entails use of a recombinant viral or non-viral vector followed by in vitro transfection of specific mammalian cell populations, recovery and purification of the transfected cells and administration to the patient. In vivo gene therapy entails the same use of vector and transfection without removing any cells or tissue from the patient. The specific cell populations utilized as targets for transfection by the recombinant vector containing a nucleic acid sequence of interest may include, but are not limited to, (1) bone marrow cell populations containing hematopoietic progenitor cells; (2) peripheral blood leukocyte populations, preferably CD34 + blood leukocyte populations, which are enriched for hematopoietic cells and can be utilized to repopulate the transfected hematopoietic cells upon introduction into the patient without ablation; (3) peripheral blood lymphocyte populations; (4) myoblast cells, which may be transplanted back into the host subsequent to in vitro transfection of the nucleic sequence of interest; and (5) delivery of the recombinant viral or non-viral vector containing the nucleic acid sequence of interest, or the nucleic acid sequence itself, by intramuscular injection.

For example, the ex vivo use of bone marrow containing hematopoietic progenitor cells containing a vector capable of encoding upon expression, a CD2 binding agent is well within the level of skill in the art. Briefly, bone marrow cells are removed, infected with the recombinant virus, and reintroduced back into the mammalian host. Thus, a gene or gene fragment is systemically distributed within the mammalian host, expression of this DNA sequence resulting in systemic delivery of the gene product within the mammalian host.

Peripheral blood is a source of mammalian cells for infection by viral mediated vectors. More specifically, blood leukocytes, especially the CD34 + population which contain the circulating hematopoietic stem cells, may be used as target cells for viral infection, followed by repopulation of the mammalian host marrow without ablation (Karlsson, et al., 1993, Bone Marrow Transplant. 11(supp. 1): 124-127). Additionally, lymphocytes may also be utilized as target cells to promote systemic delivery of the nucleic acid sequence of interest. The lymphocytes may be removed from the peripheral blood of the host, cultured by known techniques and used as the target cell population for infection by viral vectors containing the nucleic acid sequence of interest. The infected lymphocytes may then be injected into the mammalian host (e.g., see Anderson, et al., 1990, Human Gene Therapy 1: 331-361). An additional target cell population are myoblasts. Blood flow into the relatively large mass of skeletal muscle renders this tissue a localized repository for the nucleic acid sequence of interest. Therefore, in vitro infection and reintroduction of myoblast cells into the mammalian host provides a local target within the host which results in systemic delivery of the CD2 product. Myoblast culture, infection and reintroduction into the host can be achieved by known techniques (e.g., see Dai, et al., 1992, Proc. Natl. Acad. Sci. USA 89: 10892-10895).

To this end, in vivo direct injection into cells or tissue is an additional method for local delivery of the vector molecule encoding a CD2 binding agent. Direct injection ultimately results, upon expression of the gene product, in systemic delivery of a therapeutic product within the mammalian host (Wolff, et al, 1990, Science 247: 1465-1468; Raz, et al., 1993, Proc. Natl. Acad. Sci. USA 90:4523-4527). Direct injection of naked DNA, preferably a non-viral vector such as plasmid DNA, is utilized in this mode of localized delivery.

Any eukaryotic promoter and/or enhancer sequence available to the skilled artisan which is known to up-regulate expression of the nucleic acid of interest may be used in plasmid vector constructions, including but not limited to a cytomegalovirus (CMV) promoter, a Rous Sarcoma virus (RSV) promoter, a Murine Leukemia Virus (MLV) promoter, a beta -actin promoter, as well as any cell-specific eukaryotic promoter sequence that would be known to be active in the cell targeted for transduction. Moreover, any of the methods known in the art for the insertion of polynucleotide sequences into a vector may be used. See, for example, Sambrook et al., **Molecular Cloning: A Laboratory Manual,** Cold Spring Harbor Laboratory, Cold Spring Harbor, NY (1989) and Ausubel et al., **Current Protocols in Molecular Biology,** J. Wiley & Sons, NY (1992). Conventional vectors consist of appropriate transcriptional/translational control signals operatively linked to the polynucleotide sequence for a particular interferon. Promoters/enhancers may also be used to control expression of interferons. (See Section III)

Expression vectors compatible with mammalian host cells for use in gene therapy of cells include, for example, plasmids; avian, murine and human retroviral vectors; adenovirus vectors; herpes viral vectors; parvoviruses; and non-replicative pox viruses. In particular, replication-defective recombinant viruses can be generated in packaging cell lines that produce only replication-defective viruses. See **Current Protocols in Molecular Biology:** Sections 9.10-9.14 (Ausubel et al., eds.), Greene Publishing Associates, 1989.

Specific viral vectors for use in gene transfer systems are now well established. See for example: Madzak et al., J. Gen . Virol., 73: 1533-36 (1992) (papovavirus SV40); Berkner et al., Curr. Top. Microbiol. Immunol., 158: 39-61 (1992) (adenovirus); Moss et al., Curr. Top. Microbiol. Immunol., 158: 25-38 (1992) (vaccinia virus); Muzyczka, Curr. Top. Microbiol. Immunol., 158: 97-123 (1992) (adeno-associated virus); Margulskee, Curr. Top. Microbiol. Immunol., 158: 67-93 (1992) (herpes simplex virus (HSV) and Epstein-Barr virus (HBV)); Miller, Curr. Top. Microbiol. Immunol., 158: 1-24 (1992) (retrovirus); Brandyopadhyay et al., Mol. Cell. Biol., 4: 749-754 (1984) (retrovirus); Miller et al., Nature, 357: 455-450 (1992) (retrovirus); Anderson, Science, 256: 808-813 (1992) (retrovirus).

Preferred vectors are DNA viruses that include adenoviruses (preferably Ad-2 or Ad-5 based vectors), herpes viruses (preferably herpes simplex virus based vectors), and parvoviruses (preferably "defective" or non-autonomous parvovirus based vectors, more preferably adeno-associated virus based vectors, most preferably AAV-2 based vectors). See, e.g., Ali et al., Gene Therapy 1: 367-384, 1994; U.S. Patent **4,797,368** and **5,399,346** and discussion below. Adenovirus vectors are capable of providing extremely high levels of transgene delivery to virtually all cell types, regardless of the mitotic state. High titers (10¹¹ plaque forming units/ml) of recombinant virus can be easily generated in 293 cells (an adenovirus-transformed, complementation human embryonic kidney cell line: ATCC CRL1573) and cryo-stored for extended periods without appreciable losses. The efficiency of this system in delivering a therapeutic transgene *in vivo* that complements a genetic imbalance has been demonstrated in animal models of various disorders. See Y. Watanabe, Atherosclerosis, 36: 261-268 (1986); K. Tanzawa et al, FEBS Letters, 118(1):81-84 (1980); J.L. Golasten et al, New Engl.J. Med., 309 (11983): 288-296 (1983); S. Ishibashi et al, J. Clin. Invest., 92: 883-893 (1993); and S. Ishibashi et al, J. Clin. Invest., 93: 1889-1893 (1994). Indeed, recombinant replication defective adenovirus encoding a cDNA for the cystic fibrosis transmembrane regulator (CFTR) has been approved for use in several human CF clinical trials. See, e.g., J. Wilson, Nature, 365: 691-692 (Oct., 21, 1993). Further support of the safety of recombinant adenoviruses for gene therapy is the extensive experience of live adenovirus vaccines in human populations.

Adeno-associated viruses (AAV) have also been employed as vectors for somatic gene therapy. AAV is a small, single-stranded (ss) DNA virus with a simple genomic organization (4.7 kb) that makes it an ideal substrate for genetic engineering. Two open reading frames encode a series of *rep* and *cap* polypeptides. *Rep* polypeptides (*rep78, rep68, rep 62 and rep 40)* are involved in replication, rescue and integration of the AAV genome. The cap proteins (VP1, VP2 and VP3) form the virion capsid. Flanking the *rep* and *cap* open reading frames at the 5' and 3' ends are 145 bp inverted terminal repeats (ITRs), the first 125 bp of which are capable of forming Y- or T-shaped duplex structures. Of importance for the development of AAV vectors, the entire *rep* and *cap* domains can be excised and replaced with a therapeutic or reporter transgene. See B.J. Carter, in **Handbook of Parvoviruses,** ed., P. Tijsser, CRC Press, pp. 155-168 (1990). It has been shown that the ITRs represent the minimal sequence required for replication, rescue, packaging, and integration of the AAV genome. High level gene expression fom AAV in mice was shown to persist for at least 1.5 years. See Xiao, Li and Samulski (1996) Journal of Virology 70, 8089-8108. Since there was no evidence of viral toxicity or a cellular host immune response, these limitations of viral gene therapy have been overcome.Fisher et al. (Nature Medicine (1997) 3, 306-312) reported stable gene expression in mice following injection into muscle of AAV. Again, the virus was safe. No cellular or humoral immune response was detected against the virus or the foreign gene product.

Animal Models: The presence of suitable animal models of the various indications (such as the well known EAE model for Multiple Sclerosis) allows testing of the CD2 binding agents and the methods described. Several animal models are described in the Examples.

### Deposits

Murine hybridoma cells and anti-LFA-3 antibodies useful in the present invention are exemplified by cultures deposited under the Budapest Treaty with American Type Culture Collection, Rockville, Maryland, U.S.A., on March 5, 1991, and identified as:

| Designation | ATCC Accession Number |
|---|---|
| 1E6 | HB 10693 |
| HC- 1B11 | HB 10694 |
| 7A6 | HB 10695 |
| 8B8 | HB 10696 |

A bacteriophage carrying a plasmid encoding transmembrane LFA-3 was deposited under the Budapest Treaty with *In Vitro* International, Inc., Linthicum, Maryland, U.S.A., on May 28, 1987 under Accession No. IVI-10133. This deposit was transferred on June 20,1991 to the American Type Culture Collection (10801 University Blvd., Manassas, Virginia 20110-2209, United States) and identified as:

| Designation | ATCC Accession No. |
|---|---|
| λHT16[λgt10/LFA-3] | 75107 |

E. coli transformed with a plasmid encoding PI-linked LFA-3 was deposited under the Budapest Treaty with *In Vitro* International, Inc. on July 22, 1988 under Accession No. IVI-10 1 80. This deposit was transferred to American Type Culture Collection (present address 1080 University Blvd., Manassas, Virginia, U.S.A.) on June 20, 1991 and identified as:

| Designation | ATCC Accession No. |
|---|---|
| p24 | 68788 |

### Sequences

The following is a summary of the sequences set forth in the Sequence Listing:
SEQ ID NO: 1 DNA sequence of transmembrane LFA-3
SEQ ID NO:2 Amino acid sequence of transmembrane LFA-3
SEQ ID NO:3 DNA sequence of PI-linked LFA-3
SEQ ID NO:4 Amino acid sequence of PI-linked LFA-3
SEQ ID NO:5 DNA sequence of CD2
SEQ ID NO:6 Amino acid sequence of CD2
SEQ ID NO:7 DNA sequence of LFA3TIP
SEQ ID NO:8 Amino acid sequence of LFA3TIP

In order that this invention may be better understood, the following examples are set forth. These examples are for purposes of illustration only, and are not to be construed as limiting the scope of the invention in any manner.

### EXAMPLE 1: Modulation of Memory Effector T lymphocytes

This example illustrates that a particular CD2 binding agent, LFA3 TIP (SEQ ID NO: 8), will selectively modulate memory effector T lymphocytes in mammalian subjects with psoriasis.

### Materials and Methods

We performed a Phase II, randomized, double-blind, parallel design, four arm, placebo-controlled investigational trial of LFA3TIP in patients with chronic plaque psoriasis. The primary study objective was to determine the relationship of clinical response to dosages ranging from 0.025 to 0.15 mg/kg body weight. At 22 sites in the United States, 229 subjects ranged 18 to 70 years were enrolled. Entry criteria included plaque psoriasis of at least one year duration, with greater than 10% surface area involvement and not controlled by topical agents. The mean duration of disease was 16 years (1-62 year range). Phototherapy and systemic therapy was discontinued one month prior to the start of the study. LFA3TIP is packaged as a frozen solution in vials containing 10 mg/mL fusion protein per vial and exipient materials (sodium chloride, monobasic potassium phosphate, and dibasic sodium phosphate). The vials are stored at -70°C (-94°F) and thawed in the refrigerator or at room temperature and diluted with 0.9% sodium chloride to a final volume of 5 mL for IV bolus administration.

The CD2 binding agent was administered once a week by i.v. bolus for 12 weeks; subjects were followed for an additional 12 weeks after the last dose. Disease severity was assessed using a Physicial Global Assessment and the Psoriasis Activity and Severity Index (PASI) with the primary efficacy endpoints assessed at 2 weeks following the last dose of the study drug.

Peripheral blood was drawn from patients and lymphocytes separated from using ficoll-hypaque gradient centrifugation. Cells (about 3 x 10⁵) were assayed by flow cytometry (FACSTAR PLUS, Becton Dickinson, San Jose, CA). Ten thousand events were monitored.

### Results

We see statistically significant differences between LFA3TIP and placebo in a number of endpoints, including reduction in PASI score in the two higher LFA3TIP dosages. Clinical response rates increased with greater concentration of LFA3TIP (data not presented). Moreover, treatment with this CD2 binding agent was associated with a reduction in peripheral CD4+ and CD8+ T lymphocytes and to natural killer cells. We observed selective reduction of CD45 RO+ memory-effector T cells over CD45RA+ naïve CD4+ and CD8+ cells. Specifically, flow cytometric analysis revealed a greater reduction in high density T cells with memory/effector phenotypes (CD4-CD45RO and CD8-CD45RO) than in low density CD2 T cells with naïve phenotypes (CD4-CD45RA and CD8-CD45RA). Figure 1 is a graph showing the selective reduction of memory effector T cells relative to naive T cells in patients with psoriasis receiving treatment with LFA3TIP. The horizontal bar above the X-axis represents the duration of chronic treatment, which was stopped at approximately 80 days. The curves represent the mean absolute lymphocyte subset counts from the 57 patients in each treatment group. There are statistically significant dose-related reductions in memory effector T cells (CD 45 RO+). No changes in peripheral naive T cells (CD45 RA+) were observed (CD45 RA+) regardless of treatment regimen. T cell counts in the various CD2 binding agent treatments show a decrease, approaching some relatively constant value, during the 80 day chronic administration period. After treatment was terminated, the memory effector T cells count began to increase again.

LFA3TIP was well tolerated with non drug-related serious adverse events reported. A transient, mild, dose-dependent reduction from pro-infusion levels in the absolute number of peripheral lymphocytes was observed in all subjects who received LFA3TIP. This reduction was noted in total, CD2, CD4, and CD8 lymphocytes, but not in CD19 lymphocytes.

### EXAMPLE II: Treatment Of Rheumatoid Arthritis

Human patients having rheumatoid arthritis (RA) are selected for treatment using a CD2 binding agent such as LFA3-TIP. This material is prepared and packaged in Example 1 as described and is administered to patients at doses of about 7.5 to 10 mg weekly for a period of 12-24 weeks. To determine optimum dose and schedule, a parallel design study using the above-referenced different regimens are performed. Peripheral blood is drawn from patients and lymphocytes separated from using ficoll-hypaque gradient centrifugation. Cells (about 3 x 10⁵) are assayed by flow cytometry (FACSTAR PLUS, Becton Dickinson, San Jose, CA), as above. Patients are monitored using several indicia, including joint swelling and tenderness scores, that are included as part of the standardized "ACR 20" studies. See Felson, D.T. et al., "American College of Rheumatology Core Set of Disease Activity Measurements for Rheumatoid Arthritis Clinical Trials", Arth. Rheum., 26: 729-740 (1993).

### Example III: Treatment of Inflammatory Bowel Disease (IBD), Crohn's Disease and Colitis

Inflammatory bowel disease is a generic term relating to a group of chronic inflammatory disorders of unknown etiology involving the gastrointestinal tract. Ulcerative colitis (UC) and Crohn's disease, the two major forms of idiopathic Inflammatory Bowel Disease (IBD) in humans, are widespread and poorly understood disorders (Kirsner, J. B., et al., eds., Inflammatory Bowel Disease: 3rd ed., Lea and Febiger, Philadelphia (1988); Goldner, F. H., et al., Idiopathic Inflammatory Bowel Disease, in Stein, J. H., ed., Internal Medicine, Little Brown & Co., Boston, pp. 369-380 (1990); Cello, J. P., et al.. Ulcerative Colitis, in Sleisenger, M. H., et al.. eds., Gastrointestinal Disease: Pathophysiology Diagnosis Management, W. B. Saunders Co., Philadelphia, p. 1435 (1989)). While the cause of inflammatory bowel disease is unknown, possible etiologic factors include infectious, immunologic, familial or psychological factors. Secondary extraintestinal manifestations such as arthritis and pericholangitis often times occur during chronic inflammatory bowel disease. Drug therapy of inflammatory bowel disease includes administration of anti-inflammatory drugs, sulfasalazine (the active compound thought to be 5-aminosalicylate, most likely by inhibiting prostaglandin synthesis) and glucocorticoids.

Crohn's Disease or Regional Ileitis, is the term applied to a condition in which there is an inflammation of an area of the small intestine. This is usually accompanied by colicky abdominal pain, irregularity of the bowels, loss of weight and slight fever. The abdomen is generally distended and the thickened intestine may be felt. The narrowed intestinal canal may become obstructed, necessitating immediate operation. The cause of the disease is unknown. The primary lesion is hyperplasia of the lymph tissue in the submucosa of the intestine and in the lymph glands. Crohn's Disease is a chronic condition of the gastrointestinal tract and infects most commonly the ileum, colon or a combination of both. It is distinguished from ulcerative colitis by a differential diagnosis.

The pathology of ulcerative colitis usually refers to a more superficial mucosal disease in contrast to Crohn's disease with its deep, often transmucosal involvement and fissures (Riddell, R. H., ed., Pathology of Drug-induced and Toxic Diseases, Churchill Livingstone, New York (1982); Morrison, B. C., et al., eds., Gastrointestinal Pathology, 2d ed., London (1979); Fenoglio-Preiser, C. M., et al., eds., Gastrointestinal Pathology: An Atlas and Text, Raven Press, New York (1989); Goldman, H., et al., Hum. Pathol. 13:981-1012 (1982)). Ulcerative colitis typically involves the rectum and extends proximally without intervening uninvolved "skip" areas which are usually the hallmarks of Crohn's disease.

The available animal models for IBD can be divided into naturally occurring and experimentally-induced animal models. Unfortunately, only a few spontaneous and rarely occurring models of intestinal inflammation due to a genetic defect are available and most of these are not idiopathic but are induced by bacteria or other infectious agents (e.g., hyperplasia, crypt abscesses, ulcers in mice with *Bacillus psyliformnis* and hamster with "rod-shaped bacteria") (Strober, W., Dig. Dis. Sci. 33 Suppl.:3S-1OS (1988)). Rare forms of spontaneous ulcerative colitis and granulomatous enterocolitis also occur in rats and horses, respectively. In addition, marmosets and the cotton-top tamarin are used as a spontaneous model of ulcerative colitis and colonic adenocarcinomas (Clapp, N. K., et al., eds., Dig. Dis. Sci. 33 Suppl.:1S-158S (1988)). Experimentally induced animal models of ulcerative colitis are usually produced by exposure to toxic dietary substances, pharmacologic agents or other environmental chemicals, or by administration of materials derived from patients, or by manipulation of the animal's immune system (Strober, W., Dig. Dis. Sci. 33 Suppl.:3S-10S (1988); Beekan, W. L., Experimental inflammatory bowel disease, in: Kirsner, J. B., et al., eds., Inflammatory Bowel Disease, Lea and Febiger, Philadelphia, pp. 37-49 (1988); Onderdonk, A. B., Dig. Dis. Sci. 33 Suppl.:40S-44S (1988)).

One can examine the potential therapeutic efficacy and mechanisms of action of a CD2 binding agent in the treatment of Crohn's disease using a cotton-top tamarin model. See, for example, J. Madara et al., "Characterization of Spontaneous Colitis in Cotton-Top Tamarin *(Saguinus oedipus)* and Its Response to Sulfasalazine," Gastroenterology, 88, pp. 13-19 (1985). A stock solution in sterile saline of CD2 binding agent (e.g., LFA3TIP) and a placebo control (saline only), are prepared for administration to ten cotton top tamarins (CTTs) exhibiting symptoms of spontaneous colitis (i.e., diarrhea, etc.; see, Madara et al., supra). Five CTTs receive CD2 binding agent and five receive placebo, by intravenous injection. The CTTs receiving CD2 binding agent are injected with 1 mg agent per day (i.e., about 2 mg/kg/day, based on approximate half-kilogram weight of a CTT) for eight days (on Days 0, 1, 2, 3, 4, 5, 6, and 7 of the trial). Colon tissue samples obtained from the animals are biopsied every other day (on Days 0, 2, 4, 6, 8, and 10 of the trial). Data from the biopsies are used to determine an acute inflammation index for each animal, giving a semi-quantitative analysis of the course of the colitis. (See, Madara et al supra.) Peripheral blood is drawn and lymphocytes separated from using ficoll-hypaque gradient centrifugation. Cells (about 3 x 10⁵) are assayed by flow cytometry (FACSTAR PLUS, Becton Dickinson, San Jose, CA), as above. Results are expected to show selective reduction of memory effector T cells and show that treatment with CD2 binding agent results in a significant (p < 0.01) decrease in acute inflammation index.

In humans, patients having IBD are selected for treatment using a CD2 binding agent such as LFA3-TIP. This material is prepared and packaged in Example 1 as described and is administered to patients at doses of about 7.5 to 10 mg weekly for a period of 12-24 weeks. To determine optimum dose and schedule, a parallel design study using the above-referenced different regimens are performed. Peripheral blood is drawn from patients and lymphocytes separated from using ficoll-hypaque gradient centrifugation. Cells (about 3 x 10⁵) are assayed by flow cytometry (FACSTAR PLUS, Becton Dickinson, San Jose, CA), as above. Patients are monitored using several indicia, including the Crohn's Disease Activity Index (Kjeldsen,J. and Schaffalitzky de Muckadell, O.B., Scand. J. Gastroenterol. 28: 1-9 (1993).

### Example IV: Treatment of Uveitis

Uveitis is a disease of the eye which can be located throughout the eye including the posterior and anterior chambers of the eye as well as the vitreous body. Uveitis, the inflammation of the uvea, is responsible for about 10% of the visual impairment in the United States. Panuveitis refers to inflammation of the entire uveal (vascular) layer of the eye. Posterior uveitis generally refers to chorioretinitis and anterior uveitis refers to iridocyclitis. The inflammatory products, that is, cells, fibrin, excess protein, of these inflammations are commonly found in the fluid spaces of the eye including the anterior chamber, posterior chamber and vitreous space as well as the tissue involved in the inflammatory response. Uveitis may occur following surgical or traumatic injury to the eye, as a component of an autoimmune disorder such as rheumatoid arthritis, Behcet's disease, ankylosing spondylitis, sarcoidosis, as an isolated immune mediated ocular disorder, i.e., pars planitis, iridodyclitis etc., unassociated with known etiologies, and following certain systemic diseases which cause antibody-antigen complexes to be deposited in uveal tissues. Together these disorders represent non-infectious uveitities.

### Treatment in Mammals

Three cynomologous monkeys are used. Animals are anesthetized using ketamine (30 mg/kg) for all procedures. Devices containing 6 mg of LFA3-TIP are implanted into the right eye and control devices consisting of polymer alone are implanted into the left eye as described. The monkey has a well developed pars plana so cryopexy is unnecessary. All devices are implanted 3 mm posterior to the limbus in the inferior temporal quadrant. Animals are anesthetized at 1 week, 2 weeks, 4 weeks, 2 months, 4 months, and 6 months and undergo examination with indirect ophthalmoscopy and electroretinography. Electroretinography is performed as described except that averages of 5 responses are acquired with a Nicolet CA-1000 clinical averager (Madison, Wis.) and a CKA Ganzfeld flash (Gaithersburg, Md.). At 6 months a sample of blood is analyzed by HPLC for cyclosporine (detection limit = 25 ng/ml). After 6 months the animals are sacrificed with an overdose of pentobarbital and then were immediately perfused via the left ventricle with 10% buffered formalin. Eyes are placed in fixative and then subsequently embedded in paraffin, sectioned and examined in a masked fashion for evidence of toxicity. Peripheral blood was drawn and lymphocytes separated from using ficoll-hypaque gradient centrifugation. Cells (about 3 x 10⁵) were assayed by flow cytometry (FACSTAR PLUS, Becton Dickinson, San Jose, CA).

### Pharmacokinetics

Twenty four animals (Group 1) were used. CD2 binding agent is mixed into stock solutions of 50:50 ethanol:water at a concentration of 1 ug/20 ul and 10 ug/20 ul. Tritiated CD2 binding agent (1 uCi/20 ml) was also added to the stock solution. Animals are anesthetized with ketamine (60 mg) and xylazine (20 mg) and the pupils are dilated with one drop of phenylephrine hydrochloride (2.5%) and tropicamide (1%). An intravitreal injection of CD2 binding agent is then given through the superior rectus muscle approximately 5 mm posterior to the limbus using a 30 gauge needle. Twelve animals receive a dose of 1 ug in 20 ul and 12 animals received a dose of 10 ug in 20 ul). The injection is given into the midvitreous cavity and care is taken to avoid the lens. For the 1 ug group, 3 animals are sacrificed at 0.5, 3, 6 and 24 hours. For the 10 ug group, 3 animals are sacrificed at 0.5, 6, 24 and 48 hours. The eyes are enucleated and then immediately frozen at - 70o C. Tissues are prepared for assay as described below. Intravitreal half-life and volume of distribution are determined from regression analysis of a 1 n concentration versus time plot.

### Treatment

Eleven animals are used. CD2 binding agent contained in a sustained release device is inserted into the right eye. A device consisting of polymers only is inserted into the left eye. Prior to device insertion an examination consisting of ophthalmoscopy and electroretinography (ERG) was performed. Scotopic and photopic electroretinograms (ERG's) is recorded from both eyes using contact lens electrodes (ERG-Jet) with a two-channel clinical signal averager (Cadwell 5200, Kennewick, Wash.) and a Ganzfeld flash unit (Cadwell VPA-10, Kennewick, Wash.). Dark adapted ERGs, performed after at least 30 minutes of dark adaptation, are elicited at 0.33 Hz and 20 stimulus presentations are averages. Light adapted ERGs were performed at 2.8 Hz after at least 5 minutes of light adaptation and are also the average of 20 stimuli. Resultant waves are evaluated for amplitude and latency. To minimize the effect of individual and daily variation (13-15) the ERG responses are evaluated using a ratio of the amplitude of the experimental (right) to the amplitude of the control (left). When the amplitudes of the experimental and control eyes are equal, the ratio equals 1. A decrease in the ratio reflects a relative decrease in the amplitude of the experimental eye.

Examinations are repeated on days 1, 7, 14 and then every two weeks for the next 6 months. Three animals are sacrificed at 6 weeks and two animals are sacrificed at 16 weeks. To assess for reversible toxicity the device is removed from 3 animals at 16 weeks. These animals are examined weekly for two months and then sacrificed. The remaining two animals are sacrificed at 26 weeks. Following sacrifice, the animals are perfused with fixative (either 10% buffered formalin or 6% phosphate buffered glutaraldehyde) via the left ventricle. The eyes are enucleated and placed in fixative. Three 3 mm by 6 mm specimens from the posterior pole are then embedded in either paraffin or plastic and sectioned. One section from each specimen is examined in a masked manner for evidence of toxicity.

### EXAMPLE V. Treatment of Psoriatic Arthritis

This example provides a protocol to test the clinical effects and tissue response of a CD2 binding agent in patients with psoriatic arthritis.

Psoriatic arthritis occurs in 5-8% of patients with psoriasis and is a common chronic rheumatic disease that may result in considerable joint damage if left untreated. Recent clinical studies have focused on the identification of poor prognostic factors. Early aggressive treatment is indicated in patients with significant joint inflammation and certain HLA antigens. Psoriasis and psoriatic arthritis are considered typical T cell mediated diseases. Immunopathogenic evidence continues to point toward an HLA class I mediated disease with perhaps an important role for CD8+ T cells.

### STUDY DESIGN

This is a Phase II pilot study of an exemplary CD2 binding agent in patients with psoriatic arthritis.
The study will consist of a 3 month treatment period with weekly intravenous injections of a CD2 binding agent. The effects on psoriatic skin lesions and on arthritis will be measured by clinical and routine laboratory methods at the start, during and after the treatment period. Directly preceding, after 4 weeks and following the treatment period synovial biopsies will be taken for immunohistologic investigations using standardized methods. Preceding, after 4 and 12 weeks and at the end of a 3 months post-treatment period *skin biopsies* will be taken for immunohistologic investigations using standardized methods.

### STUDY POPULATION

### Number of Subjects

Patients with psoriatic arthritis are screened in order to select patients for the study if all of the following inclusion criteria are met:
1) Male or female patients between 18 and 70 years, inclusive.
2) Female patients must be of non-childbearing potential (surgically sterile or at least one year post-menopausal) or have a negative pregnancy test at the screening visit and maintain an effective birth control method at least one month prior to study drug administration, during study and in a period of 6 months post-dosing.
3) Patients must fulfil the criteria for diagnosis of definite psoriatic arthritis
4) Patients must have clinically apparent arthritis of at least one knee joint and 3 other joints, morning stiffness of at least 30 minutes and an erythrocyte sedimentation rate (ESR) of at least 28 mm.
5) Patients must have a diagnosis of plaque-type psoriasis greater then 12 months prior to the first dose of study drug.
6) Patients must have a PASI of 12 or greater.
7) Patients must have discontinued all immunomodulating medication such as methotrexate, cyclosporin and corticosteroids and all other compounds used to treat psoriasis and/or psoriatic arthritis except non steroidal anti-inflammatory drugs (NSAIDs), at least four weeks prior to the Screening Visit
8) Patients must have an absolute CD4+ lymphocyte count at or greater than the lower limit of normal within 14 days prior to the first dose.

### Treatment Schedule

The CD2 binding agent will be administered as an intravenous bolus of 7.5 mg fixed dose, given once every week for a total of twelve doses.

### Modification of Treatment Schedule

In order for a subject to receive the next dose of study drug, the following must occur:
- The absolute number of peripheral lymphocytes obtained for each subject at the site within 24 hours of administration, must be greater than 67% of the lower limit of normal OR greater than 50% of the subject's baseline measure.
- The absolute CD4⁺ lymphocytes obtained prior to the previous dose must be greater than 300 cells/mm³.
If any one of the above criteria is not met, the subject's dose will be withheld and the subject will return the following week for repeat evaluation. If any subject experiences a sustained reduction in absolute number of peripheral lymphocytes or absolute CD4⁺ lymphocytes for more than 28 days, study drug will be permanently discontinued.

### Discontinuation of Study Drug and Subjects

The following sections describe the criteria for discontinuing study drug, and the criteria for withdrawing from the study.

### SCHEDULE OF EVENTS

### Tests and Evaluations

All blood samples for hematology and lymphocyte subset analysis will be collected at the same time of the day in order to prevent artifact of diurnal variation.

The following tests and evaluations are to be conducted at the time points indicated prior to each dose of study drug, unless otherwise specified:
- A complete physical examination to be performed at Visits 5, 9, and 13.
- Measurement of vital signs to be performed at Visits 1 through 13 and Visits 15 through 17.
- Recording of body weight to be performed at Visits 1 through 13 and Visits 15 through 17.
- Urinalysis to be performed at Visits 5, 9, 13 and 17.
- Blood chemistry to be performed at Visits 5, 9, 13 and 17.
- Hematology to be performed at Visits 1 through 17. The first lab test to be performed within 48 hours prior to the first dose of study drug. All other tests to be performed within 24 hours prior to each dose.
- Lymphocyte subset analysis to be performed at Visits 1 through 17.
- Pregnancy (urine) test for women to be performed at Visits 5, 13 and 17.
- Quantitative measurement of immunoglobulins IgG, IgA, and IgM to be performed at Visit 13.
- PASI to be performed at Visits 1, 3, 5, 7, 9, 11 and 13 through 17.
- Nail evaluation to be performed at Visits 1, 3, 5, 7, 9, 11 and 13 through 17.
- Measuring of sIL-2R and sCD27 at Visits 1, 3, 5, 7, 9, 11 and 13 through 17.
- Skin punch biopsy of involved, non-target lesion from an area not exposed to sunlight to be performed at Visits 1, 5, 13 with an optional biopsy at Visit 17.
- ACR Core Set to be evaluated at Visits 1, 5, 9, 13 and 17.
- ACR Improvement Criteria to be evaluated at Visits 5, 9, 13 and 17.
- Knee arthroscopic biopsy of synovium to be performed at Visits -1, 5 and 13.

### EFFICACY ASSESSMENTS

### Clinical Efficacy Assessments

### Arthritis

All efficacy assessments on arthritis are to be performed by the same investigator/rheumatologist for each subject:
- ACR Core Set and Improvement Measurements

### Skin lesions

All efficacy assessments on skin lesions are to be performed by the same investigator/dermatologist for each subject:
- PASI score
- Nail evaluation

### Laboratory Efficacy Assessments

- Arthroscopic synovial biopsy
- Immunohistological studies of synovium
- Serum disease activity markers for psoriasis
- Skin biopsy
- Immunohistological studies of the skin

### SAFETY ASSESSMENTS

### Clinical Safety Assessments

- Physical examination.
- Vital signs (temperature, heart rate, respiration rate, and supine blood pressure).
- Body weight.
- Monitoring for infection.
- Monitoring adverse events.

### Laboratory Safety Assessments

- Hematology: complete blood count with differential and platelet count will be performed within 24 hours of dosing and monitored by the investigator at each site.
- Blood chemistry profile: sodium, potassium, chloride, bicarbonate, blood urea nitrogen, creatinine, calcium, phosphate, albumin, total protein, alkaline phosphatase, total bilirubin, ALAT, ASAT, and gamma glutamyl transaminase.
- Urinalysis.

### Product/Trial Specific Safety Assessments

- Peripheral lymphocyte subset quantitation using flow cytometry with well defined subset markers for T lymphocytes (CD4, CD8).

### STATISTICAL STATEMENT AND ANALYTICAL PLAN

### Sample Size Considerations

A pilot study with 10 subjects is considered appropriate for evaluation of safety and efficacy. Past experience with CD2 binding agents such as LFA-3/IgG₁ fusion protein has demonstrated consistent effects on total lymphocytes, and CD4 and CD8 subsets.

### Description of Endpoints

The primary clinical efficacy endpoints for arthritis and psoriasis are based on the end of treatment after 12 weeks.Surrogate endpoints for clinical efficacy are based on 4 weeks and 8 weeks of treatment. The primary safety endpoint is based on the end of a post treatment observation period of 3 months. Surrogate endpoints for immunohistological changes in skin and synovium are based on two points in time: after 4 weeks and after 12 weeks of treatment.

### Statistical Methods To Be Used in Objective Analyses

The general method of analysis will be to establish differences of clinical and laboratory parameters between baseline and the end of therapy. This will be done using, for example, a one-way analysis of variance or covariance, or logistic regression. Responses may need to be transformed prior to analysis. Non-parametric techniques will be used when appropriate.

### Clinical Efficacy Analyses

The proportion of subjects demonstrating 20% improvement in ACR Core Set Measurements will be determined. The proportion of subjects with at least a 50% decrease from baseline in their PASI score will be determined. Other efficacy measures include minimum PASI score, total erythema score, total induration score, total desquamation score and nail score.

### Laboratory Efficacy Analyses

Immunohistological studies of synovium and skin and serum levels of psoriasis activity markers will provide data that will be compared with baseline values.

While we have described a number of embodiments of this invention, it is apparent that our basic embodiments can be altered to provide other embodiments that utilize the processes of this invention. Therefore, it will be appreciated that the scope of this invention includes all alternative embodiments and variations which are defined in the foregoing specification and by the claims appended hereto; and the invention is not to be limited by the specific embodiments that have been presented herein by way of example.

## Claims

1. Use of an effective amount of a CD2 binding agent selected from the group of anti-LFA-3 antibody homologs, soluble LFA-3 polypeptides, soluble CD2 polypeptides, CD2 mimetic agents, and LFA-3 mimetic agents for the preparation of a pharmaceutical composition for selectively reducing memory effector CD45RO-positive T lymphocytes in a subject, thereby treating a medical condition, the condition being selected from the group consisting of psoriatic arthritis, multiple sclerosis, atopic dermatitis, uveitis, inflammatory bowel disease, Crohn's disease, ulcerative colitis and cutaneous T cell lymphoma.

2. Use of an effective amount of a CD2 binding agent selected from the group of anti-LFA-3 antibody homologs, soluble LFA-3 polypeptides, soluble CD2 polypeptides, CD2 mimetic agents, and LFA-3 mimetic agents, at a dosage from 0.001 to 50 mg per kg body weight of a subject, for the preparation of a pharmaceutical composition for selectively reducing memory effector CD45RO-positive T lymphocytes in a subject, thereby treating a medical condition, the condition being selected from the group consisting of psoriatic arthritis, rheumatoid arthritis, multiple sclerosis, atopic dermatitis, uveitis, inflammatory bowel disease, Crohn's disease, ulcerative colitis and cutaneous T cell lymphoma.

3. The use of claim 1 or 2, wherein the CD2 binding agent is a soluble LFA-3 polypeptide.

4. The use of claim 3, wherein the CD2 binding agent is a soluble LFA-3 polypeptide comprising an amino acid sequence that is selected from the group consisting of:
(a) amino acid 1 to amino acid 92 of SEQ ID NO: 2;
(b) amino acid 1 to amino acid 80 of SEQ ID NO: 2;
(c) amino acid 50 to amino acid 65 of SEQ ID NO: 2; and
(d) amino acid 20 to amino acid 80 of SEQ ID NO: 2.

5. The use of claim 4, wherein the soluble LFA-3 polypeptide is a fusion protein comprising the amino terminal 92 amino acids of mature LFA3 and the C-terminal 10 amino acids of a human IgG1 hinge region.

6. The use of claim 5, wherein the soluble LFA-3 fusion protein further comprises the CH2 and CH3 regions of a human IgG1 heavy chain constant domain.

7. A population of memory effector T lymphocytes obtainable from a mammal having a condition **characterized by** the presence of infiltrating memory effector T lymphocytes in an organ of the mammal, wherein the T cell population is in combination with a CD2 binding agent.

8. The population of cells of claim 7, wherein the CD2 binding agent is a soluble LFA-3 polypeptide comprising an amino acid sequence that is selected from the group consisting of:
(a) amino acid 1 to amino acid 92 of SEQ ID NO: 2;
(b) amino acid 1 to amino acid 80 of SEQ ID NO: 2;
(c) amino acid 50 to amino acid 65 of SEQ ID NO: 2; and
(d) amino acid 20 to amino acid 80 of SEQ ID NO: 2.

9. The population of cells of claim 8, wherein the soluble LFA-3 polypepti is a fusion protein comprising the amino terminal 92 amino acids of mature LFA-3 and the C-terminal 10 amino acids of a human IgG1 hinge region.

10. The population of cells of claim 9, wherein the soluble LFA-3 fusion protein further comprises the CH2 and CH3 regions of a human IgG1 heavy chain constant domain.

11. The population of cells of any one of claims 7 to 9, wherein the cells are obtainable from a mammal having a condition selected from the group consisting of psoriatic arthritis, rheumatoid arthritis, multiple sclerosis, atopic dermatitis, uveitis, inflammatory bowel disease, Crohn's disease, ulcerative colitis, and cutaneous T cell lymphoma.

12. The use of any one of claims 1 to 6, wherein the condition is uveitis.

13. The use of any one of claims 1 to 6, wherein the condition is inflammatory bowel disease.

14. The use of any one of claims 1 to 6, wherein the condition is cutaneous T cell lymphoma.

15. The use of any one of claims 1 to 6, wherein the condition is Crohn's disease.

16. The use of any one of claims 1 to 6, wherein the condition is ulcerative colitis.

17. The use of any one of claims 1 to 6, wherein the condition is multiple sclerosis.

18. The use of any one of claims 1 to 6, wherein the condition is psoriatic arthritis.

19. The use of any one of claims 1 to 6, wherein the condition is atopic dermatitis.

20. The use of claim 1, wherein the dosage of the CD2 binding agent ranges from 0.001 to 50 mg binding agent per kg body weight.

21. The use of claim 20, wherein the dosage of the CD2 binding agent ranges from 0.025 to 0.15 mg binding agent per kg body weight.

22. The use of claim 20, wherein the dosage of the CD2 binding agent is selected from 0.05, 0.075, 0.1125 and 0.165 mg binding agent per kg body weight.

23. The use of any one of claims 1 to 6 or 12 to 22, wherein the subject is a human.

24. The use of a soluble LFA-3 polypeptide and methotrexate or cyclosporin A for the preparation of a pharmaceutical composition for the treatment of rheumatoid arthritis in a subject.

25. The use of claim 24, wherein the LFA-3 polypeptide and methotrexate or cyclosporin A are formulated in single dosage form.

26. The use of claim 24, wherein the LFA-3 polypeptide and methotrexate or cyclosporin A are to be administered concurrently.

27. The use of claim 24, wherein the LFA-3 polypeptide and methotrexate or cyclosporin A are to be administered sequentially.

28. The use of claim 24, wherein the LFA-3 polypeptide and methotrexate or cyclosporin A are to be administered conjugated together.

29. The use of claim 24, wherein the LFA-3 polypeptide is formulated to be administered at a dosage ranging from 0.001 to 50 mg per kg body weight.

30. The use of claim 24, wherein the soluble LFA-3 polypeptide comprises an amino acid sequence that is selected from the group consisting of:
(a) amino acid 1 to amino acid 92 of SEQ ID NO: 2;
(b) amino acid 1 to amino acid 80 of SEQ ID NO: 2;
(c) amino acid 50 to amino acid 65 of SEQ ID NO: 2; and
(d) amino acid 20 to amino acid 80 of SEQ ID NO: 2.

31. The use of claim 30, wherein the soluble LFA-3 polypeptide is a fusion protein comprising the amino terminal 92 amino acids of mature LFA-3 and the C-terminal 10 amino acids of a human IgG 1 hinge region.

32. The use of claim 31, wherein the soluble LFA-3 fusion protein further comprises the CH2 and CH3 regions of a human IgG1 heavy chain constant domain.

33. The use of a soluble LFA-3 polypeptide and methotrexate or cyclosporin A for the preparation of a pharmaceutical composition for the treatment of psoriatic arthritis, multiple sclerosis, uveitis, inflammatory bowel disease, Crohn's disease, and ulcerative colitis in a subject.

34. The use of claim 33, wherein the soluble LFA-3 polypeptide is a fusion protein comprising the amino terminal 92 amino acids of mature LFA-3 and the C-terminal 10 amino acids of a human IgG1 hinge region.

35. The use of claim 34, wherein the soluble LFA-3 fusion protein further comprises the CH2 and CH3 regions of a human IgG1 heavy chain constant domain.

36. The use of any one of claims 2 or 3 to 6 as far as they refer back to claim 2, wherein the condition is rheumatoid arthritis.

37. The use of any one of claims 1, 2, 18, 24 or 33, wherein the agent is LFA3TIP (amino acids 1 to 319 of SEQ ID NO:8).

## Patentansprüche

1. Verwendung einer wirksamen Menge eines CD2-bindenden Agens, ausgewählt aus der Gruppe von Anti-LFA-3-Antikörper-Homologen, löslichen LFA-3-Polypeptiden, löslichen CD2-Polypeptiden, CD2-mimetischen Agentien und LFA-3-mimetischen Agentien für die Herstellung eines Arzneimittels zur selektiven Verringerung von Gedächtnis-Effektor-CD45RO-positiven T-Lymphocyten in einem Individuum, zum Behandeln eines medizinischen Zustands, wobei der Zustand ausgewählt ist aus der Gruppe bestehend aus psoriatischer Arthritis, Multipler Sklerose, atopischer Dermatitis, Uveitis, entzündlicher Darmkrankheit, Morbus Crohn, ulcerativer Colitis und kutanem T-Zell-Lymphom.

2. Verwendung einer wirksamen Menge eines CD2-bindenden Agens, ausgewählt aus der Gruppe von Anti-LFA-3-Antikörper-Homologen, löslichen LFA-3-Polypeptiden, löslichen CD2-Polypeptiden, CD2-mimetischen Agentien und LFA-3-mimetischen Agentien mit einer Dosierung von 0,001 bis 50 mg pro kg Körpergewicht eines Individuums für die Herstellung eines Arzneimittels zur selektiven Verringerung von Gedächtnis-Effektor-CD45RO-positiven T-Lymphocyten in einem Individuum, zum Behandeln eines medizinischen Zustands, wobei der Zustand ausgewählt ist aus der Gruppe bestehend aus psoriatischer Arthritis, rheumatoider Arthritis, Multipler Sklerose, atopischer Dermatitis, Uveitis, entzündlicher Darmkrankheit, Morbus Crohn, ulcerativer Colitis und kutanem T-Zell-Lymphom.

3. Verwendung nach Anspruch 1 oder 2, wobei das CD2-bindende Agens ein lösliches LFA-3-Polypeptid ist.

4. Verwendung nach Anspruch 3, wobei das CD2-bindende Agens ein lösliches LFA-3-Polypeptid ist, umfassend eine Aminosäuresequenz, die ausgewählt ist aus der Gruppe bestehend aus:
(a) Aminosäure 1 bis Aminosäure 92 von SEQ ID NO: 2;
(b) Aminosäure 1 bis Aminosäure 80 von SEQ ID NO: 2;
(c) Aminosäure 50 bis Aminosäure 65 von SEQ ID NO: 2; und
(d) Aminosäure 20 bis Aminosäure 80 von SEQ ID NO: 2.

5. Verwendung nach Anspruch 4, wobei das lösliche LFA-3-Polypeptid ein Fusionsprotein ist, umfassend die aminoterminalen 92 Aminosäuren des reifen LFA-3 und die C-terminalen 10 Aminosäuren einer menschlichen IgG1-Gelenkregion.

6. Verwendung nach Anspruch 5, wobei das lösliche LFA-3-Fusionsprotein zusätzlich die CH2- und CH3-Regionen einer konstanten Domäne der schweren Kette von menschlichem IgG1 umfasst.

7. Population von Gedächtnis-Effektor-T-Lymphocyten, erhältlich aus einem Säuger mit einem Zustand, der **gekennzeichnet ist durch** die Anwesenheit von infiltrierenden Gedächtnis-Effektor-T-Lymphocyten in einem Organ des Säugers, wobei die T-Zell-Population in Kombination mit einem CD2-bindenden Agens ist.

8. Population von Zellen nach Anspruch 7, wobei das CD2-bindende Agens ein lösliches LFA-3-Polypeptid ist, umfassend eine Aminosäuresequenz, die ausgewählt ist aus der Gruppe bestehend aus:
(a) Aminosäure 1 bis Aminosäure 92 von SEQ ID NO: 2;
(b) Aminosäure 1 bis Aminosäure 80 von SEQ ID NO: 2;
(c) Aminosäure 50 bis Aminosäure 65 von SEQ ID NO: 2; und
(d) Aminosäure 20 bis Aminosäure 80 von SEQ ID NO: 2.

9. Population von Zellen nach Anspruch 8, wobei das lösliche LFA-3-Polypetid ein Fusionsprotein ist, umfassend die aminoterminalen 92 Aminosäuren des reifen LFA-3 und die C-terminalen 10 Aminosäuren einer menschlichen IgG1-Gelenkregion.

10. Population von Zellen nach Anspruch 9, wobei das lösliche LFA-3-Fusionsprotein zusätzlich die CH2- und CH3-Regionen einer konstanten Domäne der schweren Kette von menschlichem IgG1 umfasst.

11. Population von Zellen nach einem der Ansprüche 7 bis 9, wobei die Zellen erhältlich sind aus einem Säuger mit einem Zustand ausgewählt aus der Gruppe bestehend aus psoriatischer Arthritis, rheumatoider Arthritis, Multipler Sklerose, atopischer Dermatitis, Uveitis, entzündlicher Darmkrankheit, Morbus Crohn, ulcerativer Colitis und kutanem T-Zell-Lymphom.

12. Verwendung nach einem der Ansprüche 1 bis 6, wobei der Zustand Uveitis ist.

13. Verwendung nach einem der Ansprüche 1 bis 6, wobei der Zustand entzündliche Darmkrankheit ist.

14. Verwendung nach einem der Ansprüche 1 bis 6, wobei der Zustand kutanes T-Zell-Lymphom ist.

15. Verwendung nach einem der Ansprüche 1 bis 6, wobei der Zustand Morbus Crohn ist.

16. Verwendung nach einem der Ansprüche 1 bis 6, wobei der Zustand ulcerative Colitis ist.

17. Verwendung nach einem der Ansprüche 1 bis 6, wobei der Zustand Multiple Sklerose ist.

18. Verwendung nach einem der Ansprüche 1 bis 6, wobei der Zustand psoriatische Arthritis ist.

19. Verwendung nach einem der Ansprüche 1 bis 6, wobei der Zustand atopische Dermatitis ist.

20. Verwendung nach Anspruch 1, wobei die Dosierung des CD2-bindenden Agens von 0,001 bis 50 mg bindendem Agens pro kg Körpergewicht reicht.

21. Verwendung nach Anspruch 20, wobei die Dosierung des CD2-bindenden Agens von 0,025 bis 0,15 mg bindendem Agens pro kg Körpergewicht reicht.

22. Verwendung nach Anspruch 20, wobei die Dosierung des CD2-bindenden Agens ausgewählt ist aus 0,05, 0,075, 0,1125 und 0,165 mg bindendem Agens pro kg Körpergewicht.

23. Verwendung nach einem der Ansprüche 1 bis 6 oder 12 bis 22, wobei das Individuum ein Mensch ist.

24. Verwendung eines löslichen LFA-3-Polypeptids und von Methotrexat oder Cyclosporin A für die Herstellung eines Arzneimittels zur Behandlung von rheumatoider Arthritis in einem Individuum.

25. Verwendung nach Anspruch 24, wobei das LFA-3-Polypeptid und Methotrexat oder Cyclosporin A in einer Einzeldosierungsform formuliert sind.

26. Verwendung nach Anspruch 24, wobei das LFA-3-Polypeptid und Methotrexat oder Cyclosporin A gleichzeitig zu verabreichen sind.

27. Verwendung nach Anspruch 24, wobei das LFA-3-Polypeptid und Methotrexat oder Cyclosporin A nacheinander zu verabreichen sind.

28. Verwendung nach Anspruch 24, wobei das LFA-3-Polypeptid und Methotrexat oder Cyclosporin A miteinander konjugiert zu verabreichen sind.

29. Verwendung nach Anspruch 24, wobei das LFA-3-Polypeptid formuliert ist, um in einer Dosierung, die von 0,001 bis 50 mg pro kg Körpergewicht reicht, verabreicht zu werden.

30. Verwendung nach Anspruch 24, wobei das lösliche LFA-3-Polypeptid eine Aminosäuresequenz umfasst, die ausgewählt wird aus der Gruppe bestehend aus:
(a) Aminosäure 1 bis Aminosäure 92 von SEQ ID NO: 2;
(b) Aminosäure 1 bis Aminosäure 80 von SEQ ID NO: 2;
(c) Aminosäure 50 bis Aminosäure 65 von SEQ ID NO: 2; und
(d) Aminosäure 20 bis Aminosäure 80 von SEQ ID NO: 2.

31. Verwendung nach Anspruch 30, wobei das lösliche LFA-3-Polypeptid ein Fusionsprotein ist, umfassend die aminoterminalen 92 Aminosäuren des reifen LFA-3 und die C-terminalen 10 Aminosäuren der menschlichen IgG1-Gelenkregion.

32. Verwendung nach Anspruch 31, wobei das lösliche LFA-3-Fusionsprotein zusätzlich die CH2- und CH3-Regionen einer konstanten Domäne der schweren Kette von menschlichem IgG1 umfasst.

33. Verwendung eines löslichen LFA-3-Polypeptids und von Methotrexat oder Cyclosporin A für die Herstellung eines Arzneimittels zur Behandlung von psoriatischer Arthritis, Multipler Sklerose, Uveitis, entzündlicher Darmkrankheit, Morbus Crohn und ulcerativer Colitis in einem Individuum.

34. Verwendung nach Anspruch 33, wobei das lösliche LFA-3-Polypeptid ein Fusionsprotein ist, umfassend die aminoterminalen 92 Aminosäuren des reifen LFA-3 und die C-terminalen 10 Aminosäuren der menschlichen IgG1-Gelenkregion.

35. Verwendung nach Anspruch 34, wobei das lösliche LFA-3-Fusionsprotein zusätzlich die CH2- und CH3-Regionen einer konstanten Domäne der schweren Kette von menschlichem IgG1 umfasst.

36. Verwendung nach einem der Ansprüche 2 oder 3 bis 6, sofern sie sich auf Anspruch 2 zurückbeziehen, wobei der Zustand rheumatoide Arthritis ist.

37. Verwendung nach einem der Ansprüche 1, 2, 18, 24 oder 33, wobei das Agens LFA-3TIP (Aminosäuren 1 bis 319 von SEQ ID NO: 8) ist.

## Revendications

1. Utilisation d'une quantité efficace d'un agent liant CD2 choisi dans le groupe des homologues d'anticorps anti-LFA-3, des polypeptides LFA-3 solubles, des polypeptides CD2 solubles, des agents mimant CD2 et des agents mimant LFA-3, pour la préparation d'une composition pharmaceutique pour réduire de manière sélective chez un sujet les lymphocytes T effecteurs de la mémoire CD45RO positifs, pour traiter ainsi une condition médicale, la condition étant choisie dans le groupe constitué du rhumatisme psoriasique, de la sclérose en plaques, la dermite atopique, l'uvéite, l'affection abdominale inflammatoire, la maladie de Crohn, la recto-colite hémorragique, et le lymphome cutané à cellules T.

2. Utilisation d'une quantité efficace d'un agent liant CD2 choisi dans le groupe constitué d'homologues d'anticorps anti-LFA-3, de polypeptides LFA-3 solubles, de polypeptides CD2 solubles, d'agents mimant CD2, et des agents mimant LFA-3, à un dosage allant de 0,001 à 50 mg par kilogramme de poids corporel d'un sujet, pour la préparation d'une composition pharmaceutique pour réduire de manière sélective chez un sujet les lymphocytes T effecteurs de la mémoire CD45RO positifs, pour traiter ainsi une condition médicale, la condition étant choisie dans le groupe constitué du rhumatisme psoriasique, de la arthrite rhumatoïde, de la sclérose en plaques, la dermite atopique, l'uvéite, l'affection abdominale inflammatoire, la maladie de Crohn, la recto-colite hémorragique, et le lymphome cutané à cellules T.

3. Utilisation selon la revendication 1 ou 2, dans laquelle l'agent liant CD2 est un polypeptide LFA-3 soluble.

4. Utilisation selon la revendication 3, dans laquelle l'agent liant CD2 est un polypeptide LFA-3 soluble comprenant une séquence d'acides aminés qui est choisie dans le groupe constitué de :
(a) acide aminé 1 à acide aminé 92 de SEQ ID N° : 2 ;
(b) acide aminé 1 à acide aminé 80 de SEQ ID N° : 2 ;
(c) acide aminé 50 à acide aminé 65 de SEQ ID N° : 2 ; et
(d) acide aminé 20 à acide aminé 80 de SEQ ID N° : 2.

5. Utilisation selon la revendication 4, dans laquelle le polypeptide LFA-3 soluble est une protéine de fusion comprenant les 92 acides aminés de l'extrémité amino-terminale de LFA-3 mature et les 10 acides aminés de l'extrémité C-terminale d'une région charnière d'une IgG1 humaine.

6. Utilisation selon la revendication 5, dans laquelle la protéine de fusion LFA-3 soluble comprend en outre les régions CH2 et CH3 du domaine constant d'une chaîne lourde d'une IgG1 humaine.

7. Population de lymphocytes T effecteurs de la mémoire, susceptibles d'être obtenus à partir d'un mammifère ayant une condition **caractérisée par** la présence de lymphocytes T effecteurs de la mémoire infiltrant dans un organe du mammifère, dans laquelle la population de cellules T est en combinaison avec un agent liant CD2.

8. Population de cellules selon la revendication 7, dans laquelle l'agent liant CD2 est un polypeptide LFA-3 soluble, comprenant une séquence d'acides aminés qui est choisie dans le groupe constitué de :
(a) acide aminé 1 à acide aminé 92 de SEQ ID N° : 2 ;
(b) acide aminé 1 à acide aminé 80 de SEQ ID N° : 2 ;
(c) acide aminé 50 à acide aminé 65 de SEQ ID N° : 2 ; et
(d) acide aminé 20 à acide aminé 80 de SEQ ID N° : 2.

9. Population de cellules selon la revendication 8, dans laquelle le polypeptide LFA-3 soluble est une protéine de fusion comprenant les 92 acides aminés de l'extrémité amino-terminale de LFA-3 mature et les 10 acides aminés de l'extrémité C-terminale d'une région charnière d'une IgG1 humaine.

10. Population de cellules selon la revendication 9, dans laquelle la protéine de fusion LFA-3 soluble comprend en outre les régions CH2 et CH3 du domaine constant de la chaîne lourde d'une IgG1 humaine.

11. Population de cellules selon l'une quelconque des revendications 7 à 9, dans laquelle les cellules sont susceptibles d'être obtenues à partir d'un mammifère ayant une condition choisie dans le groupe constitué du rhumatisme psoriasique, de la arthrite rhumatoïde, de la sclérose en plaques, la dermite atopique, l'uvéite, l'affection abdominale inflammatoire, la maladie de Crohn, la recto-colite hémorragique, et le lymphome cutané à cellules T.

12. Utilisation selon l'une quelconque des revendications 1 à 6, dans laquelle la condition est l'uvéite.

13. Utilisation selon l'une quelconque des revendications 1 à 6, dans laquelle la condition est l'affection abdominale inflammatoire.

14. Utilisation selon l'une quelconque des revendications 1 à 6, dans laquelle la condition est le lymphome cutané à cellules T.

15. Utilisation selon l'une quelconque des revendications 1 à 6, dans laquelle la condition est la maladie de Crohn.

16. Utilisation selon l'une quelconque des revendications 1 à 6, dans laquelle la condition est la recto-colite hémorragique.

17. Utilisation selon l'une quelconque des revendications 1 à 6, dans laquelle la condition est la sclérose en plaques.

18. Utilisation selon l'une quelconque des revendications 1 à 6, dans laquelle la condition est le rhumatisme psoriasique.

19. Utilisation selon l'une quelconque des revendications 1 à 6, dans laquelle la condition est la dermite atopique.

20. Utilisation selon la revendication 1, dans laquelle le dosage de l'agent liant CD2 va de 0,001 à 50 mg d'agent liant par kilogramme de poids corporel.

21. Utilisation selon la revendication 20, dans laquelle le dosage de l'agent liant CD2 va de 0,025 à 0,15 mg d'agent liant par kilogramme de poids corporel.

22. Utilisation selon la revendication 20, dans laquelle le dosage de l'agent liant CD2 est choisi parmi 0,05, 0,075, 0,1125 et 0,165 mg d'agent liant par kilogramme de poids corporel.

23. Utilisation selon l'une quelconque des revendications 1 à 6, 12 à 22, dans laquelle le sujet est un humain.

24. Utilisation d'un polypeptide LFA-3 soluble et de méthotrexate ou de cyclosporine A pour la préparation d'une composition pharmaceutique pour le traitement de la arthrite rhumatoïde chez un sujet.

25. Utilisation selon la revendication 24, dans laquelle le polypeptide LFA-3 et le méthotrexate ou la cyclosporine A sont formulés sous forme d'un dosage unique.

26. Utilisation selon la revendication 24, dans laquelle le polypeptide LFA-3 et le méthotrexate ou la cyclosporine A sont à administrer de manière concomitante.

27. Utilisation selon la revendication 24, dans laquelle le polypeptide LFA-3 et le méthotrexate ou la cyclosporine A sont à administrer de manière séquentielle.

28. Utilisation selon la revendication 24, dans laquelle le polypeptide LFA-3 et le méthotrexate ou la cyclosporine A sont à administrer de manière conjuguée ensemble.

29. Utilisation selon la revendication 24, dans laquelle le polypeptide LFA-3 est formulé en vue d'une administration à un dosage allant de 0,001 à 50 mg par kilogramme de poids corporel.

30. Utilisation selon la revendication 24, dans laquelle le polypeptide LFA-3 soluble comprend une séquence d'acides aminés qui est choisie dans le groupe constitué de :
(a) acide aminé 1 à acide aminé 92 de SEQ ID N° : 2 ;
(b) acide aminé 1 à acide aminé 80 de SEQ ID N° : 2 ;
(c) acide aminé 50 à acide aminé 65 de SEQ ID N° : 2 ; et
(d) acide aminé 20 à acide aminé 80 de SEQ ID N° : 2.

31. Utilisation selon la revendication 30, dans laquelle le polypeptide LFA-3 soluble est une protéine de fusion comprenant les 92 acides aminés de l'extrémité amino-terminale de LFA-3 mature et les 10 acides aminés de l'extrémité C-terminale d'une région charnière d'une IgG1 humaine.

32. Utilisation selon la revendication 31, dans laquelle la protéine de fusion LFA-3 soluble comprend en outre les régions CH2 et CH3 du domaine constant de la chaîne lourde d'une IgG1 humaine.

33. Utilisation d'un polypeptide LFA-3 soluble et de méthotrexate ou de cyclosporine A pour la préparation d'une composition pharmaceutique pour le traitement du rhumatisme psoriasique, de la sclérose en plaques, l'uvéite, l'affection abdominale inflammatoire, la maladie de Crohn et de la recto-colite hémorragique chez un sujet.

34. Utilisation selon la revendication 33, dans laquelle le polypeptide LFA-3 soluble est une protéine de fusion comprenant les 92 acides aminés amino-terminale de LFA-3 mature et les 10 acides aminés C-terminale de la région charnière d'une IgG1 humaine.

35. Utilisation selon la revendication 34, dans laquelle la protéine de fusion LFA-3 soluble comprend en outre les régions CH2 et CH3 du domaine constant de la chaîne lourde d'une IgG1 humaine.

36. Utilisation selon l'une quelconque des revendications 2 ou 3 à 6 dans la mesure où elles font référence à la revendication 2, dans laquelle la condition est la arthrite rhumatoïde.

37. Utilisation selon l'une quelconque des revendications 1, 2, 18, 24 ou 33, dans laquelle l'agent est LFA3TIP (acides aminés 1 à 319 de SEQ ID N° : 8).


### SEQUENCE LISTING

### (1) GENERAL INFORMATION:

(i) APPLICANTS: Magilavy, Daniel
(ii) TITLE OF INVENTION: Method of Modulating Memory Effector T- Cells and Compositions
(iii) NUMBER OF SEQUENCES: 8
(iv) CORRESPONDENCE ADDRESS:
   (A) ADDRESSEE: BIOGEN, INC.
   (B) STREET: 14 Cambridge Center
   (C) CITY: Cambridge
   (D) STATE: Massachusetts
   (E) COUNTRY: USA
   (F) ZIP: 02142
(v) COMPUTER READABLE FORM:
   (A) MEDIUM TYPE: Floppy disk
   (B) COMPUTER: IBM PC compatible
   (C) OPERATING SYSTEM: PC-DOS/MS-DOS
   (D) SOFTWARE: PatentIn
(vi) CURRENT APPLICATION DATA:
   (A) APPLICATION NUMBER:
   (B) FILING DATE: 31-AUG-1999
(vii) PRIOR APPLICATION DATA:
   (A) APPLICATION NUMBER: 06/098,456
   (B) FILING DATE: 31-AUG-1999
(viii) ATTORNEY/AGENT INFORMATION:
   (A) NAME: Kaplan, Warren
   (B) REGISTRATION NUMBER: 34,199
   (C) REFERENCE/DOCKET NUMBER: A005PCT
(ix) TELECOMMUNICATION INFORMATION:
   (A) TELEPHONE: (617)679-3598
   (B) TELEFAX: (617)679-2838

### (2) INFORMATION FOR SEQ ID NO:1:

(i) SEQUENCE CHARACTERISTICS:
   (A) LENGTH: 753 base pairs
   (B) TYPE: nucleic acid
   (C) STRANDEDNESS: single
   (D) TOPOLOGY: linear
(ii) MOLECULE TYPE: cDNA
(ix) FEATURE:
   (A) NAME/KEY: CDS
   (B) LOCATION: 1..750
(ix) FEATURE:
   (A) NAME/KEY: sig_peptide
   (B) LOCATION: 1..84
(ix) FEATURE:
   (A) NAME/KEY: mat_peptide
   (B) LOCATION: 85..750
(ix) FEATURE:
   (A) NAME/KEY: misc_feature
   (B) LOCATION: 1..750
   (D) OTHER INFORMATION: /note= "Human transmembrane LFA-3"
(ix) FEATURE:
   (A) NAME/KEY: misc_feature
   (B) LOCATION: 646
   (D) OTHER INFORMATION: /note= "Transmembrane domain"
(xi) SEQUENCE DESCRIPTION: SEQ ID NO:1:
   ATG GTT GCT GGG AGC GAC GCG GGG CGG GCC CTG GGG GTC CTC AGC GTG     48
   Met Val Ala Gly Ser Asp Ala Gly Arg Ala Leu Gly Val Leu Ser Val
   -28         -25                     -20                 -15
   GTC TGC CTG CTG CAC TGC TTT GGT TTC ATC AGC TGT TTT TCC CAA CAA     96
   Val Cys Leu Leu His Cys Phe Gly Phe Ile Ser Cys Phe Ser Gln Gln
           -10                  -5                  1
   ATA TAT GGT GTT GTG TAT GGG AAT GTA ACT TTC CAT GTA CCA AGC AAT    144
   Ile Tyr Gly Val Val Tyr Gly Asn Val Thr Phe His Val Pro Ser Asn
    5                  10                  15                      20
   GTG CCT TTA AAA GAG GTC CTA TGG AAA AAA CAA AAG GAT AAA GTT GCA    192
   Val Pro Leu Lys Glu Val Leu Trp Lys Lys Gln Lys Asp Lys Val Ala
                   25                  30                  35
   GAA CTG GAA AAT TCT GAA TTC AGA GCT TTC TCA TCT TTT AAA AAT AGG    240
   Glu Leu Glu Asn Ser Glu Phe Arg Ala Phe Ser Ser Phe Lys Asn Arg
               40                  45                  50
   GTT TAT TTA GAC ACT GTG TCA GGT AGC CTC ACT ATC TAC AAC TTA ACA    288
   Val Tyr Leu Asp Thr Val Ser Gly Ser Leu Thr Ile Tyr Asn Leu Thr
           55                  60                  65
   TCA TCA GAT GAA GAT GAG TAT GAA ATG GAA TCG CCA AAT ATT ACT GAT    336
   Ser Ser Asp Glu Asp Glu Tyr Glu Met Glu Ser Pro Asn Ile Thr Asp
       70                  75                  80
   ACC ATG AAG TTC TTT CTT TAT GTG CTT GAG TCT CTT CCA TCT CCC ACA    384
   Thr Met Lys Phe Phe Leu Tyr Val Leu Glu Ser Leu Pro Ser Pro Thr
    85                 90                  95                     100
   CTA ACT TGT GCA TTG ACT AAT GGA AGC ATT GAA GTC CAA TGC ATG ATA    432
   Leu Thr Cys Ala Leu Thr Asn Gly Ser Ile Glu Val Gln Cys Met Ile
                   105                 110                 115
   CCA GAG CAT TAC AAC AGC CAT CGA GGA CTT ATA ATG TAC TCA TGG GAT    480
   Pro Glu His Tyr Asn Ser His Arg Gly Leu Ile Met Tyr Ser Trp Asp
               120                 125                 130
   TGT CCT ATG GAG CAA TGT AAA CGT AAC TCA ACC AGT ATA TAT TTT AAG    528
   Cys Pro Met Glu Gln Cys Lys Arg Asn Ser Thr Ser Ile Tyr Phe Lys
           135                 140                 145
   ATG GAA AAT GAT CTT CCA CAA AAA ATA CAG TGT ACT CTT AGC AAT CCA    576
   Met Glu Asn Asp Leu Pro Gln Lys Ile Gln Cys Thr Leu Ser Asn Pro
       150                 155                 160
   TTA TTT AAT ACA ACA TCA TCA ATC ATT TTG ACA ACC TGT ATC CCA AGC    624
   Leu Phe Asn Thr Thr Ser Ser Ile Ile Leu Thr Thr Cys Ile Pro Ser
   165                 170                 175                     180
   AGC GGT CAT TCA AGA CAC AGA TAT GCA CTT ATA CCC ATA CCA TTA GCA    672
   Ser Gly His Ser Arg His Arg Tyr Ala Leu Ile Pro Ile Pro Leu Ala
               185                 190                 195
   GTA ATT ACA ACA TGT ATT GTG CTG TAT ATG AAT GGT ATT CTG AAA TGT    720
   Val Ile Thr Thr Cys Ile Val Leu Tyr Met Asn Gly Ile Leu Lys Cys
           200                 205                 210
   GAC AGA AAA CCA GAC AGA ACC AAC TCC AAT TGA                        753
   Asp Arg Lys Pro Asp Arg Thr Asn Ser Asn
           215                 220

### (2) INFORMATION FOR SEQ ID NO:2:

(i) SEQUENCE CHARACTERISTICS:
   (A) LENGTH: 250 amino acids
   (B) TYPE: amino acid
   (D) TOPOLOGY: linear
(ii) MOLECULE TYPE: protein
(xi) SEQUENCE DESCRIPTION: SEQ ID NO:2:
   Met Val Ala Gly Ser Asp Ala Gly Arg Ala Leu Gly Val Leu Ser Val
   -28         -25                 -20                 -15
   Val Cys Leu Leu His Cys Phe Gly Phe Ile Ser Cys Phe Ser Gln Gln
           -10                 -5                  1
   Ile Tyr Gly Val Val Tyr Gly Asn Val Thr Phe His Val Pro Ser Asn
    5                  10                  15                  20
   Val Pro Leu Lys Glu Val Leu Trp Lys Lys Gln Lys Asp Lys Val Ala
                   25                  30                  35
   Glu Leu Glu Asn Ser Glu Phe Arg Ala Phe Ser Ser Phe Lys Asn Arg
               40                  45                  50
   Val Tyr Leu Asp Thr Val Ser Gly Ser Leu Thr Ile Tyr Asn Leu Thr
           55                  60                  65
   Ser Ser Asp Glu Asp Glu Tyr Glu Met Glu Ser Pro Asn Ile Thr Asp
       70                  75                  80
   Thr Met Lys Phe Phe Leu Tyr Val Leu Glu Ser Leu Pro Ser Pro Thr
   85                  90                  95                  100
   Leu Thr Cys Ala Leu Thr Asn Gly Ser Ile Glu Val Gln Cys Met Ile
                   105                 110                 115
   Pro Glu His Tyr Asn Ser His Arg Gly Leu Ile Met Tyr Ser Trp Asp
               120                 125                 130
   Cys Pro Met Glu Gln Cys Lys Arg Asn Ser Thr Ser Ile Tyr Phe Lys
           135                 140                 145
   Met Glu Asn Asp Leu Pro Gln Lys Ile Gln Cys Thr Leu Ser Asn Pro
       150                 155                 160
   Leu Phe Asn Thr Thr Ser Ser Ile Ile Leu Thr Thr Cys Ile Pro Ser
   165                 170                 175                 180
   Ser Gly His Ser Arg His Arg Tyr Ala Leu Ile Pro Ile Pro Leu Ala
                   185                 190                 195
   Val Ile Thr Thr Cys Ile Val Leu Tyr Met Asn Gly Ile Leu Lys Cys
               200                 205                 210
   Asp Arg Lys Pro Asp Arg Thr Asn Ser Asn
           215                 220

### (2) INFORMATION FOR SEQ ID NO:3:

(i) SEQUENCE CHARACTERISTICS:
   (A) LENGTH: 723 base pairs
   (B) TYPE: nucleic acid.
   (C) STRANDEDNESS: single
   (D) TOPOLOGY: linear
(ii) MOLECULE TYPE: cDNA
(ix) FEATURE:
   (A) NAME/KEY: CDS
   (B) LOCATION: 1..720
(ix) FEATURE:
   (A) NAME/KEY: sig_peptide
   (B) LOCATION: 1..84
(ix) FEATURE:
   (A) NAME/KEY: mat_peptide
   (B) LOCATION: 85..720
(ix) FEATURE:
   (A) NAME/KEY: misc_feature
   (B) LOCATION: 1..720
   (D) OTHER INFORMATION: /note= "Human PI-linked LFA-3"
(ix) FEATURE:
   (A) NAME/KEY: misc_feature
   (B) LOCATION: 568..720
   (D) OTHER INFORMATION: /note= "Signal sequence for PI-linkage"
(xi) SEQUENCE DESCRIPTION: SEQ ID NO:3:
   ATG GTT GCT GGG AGC GAC GCG GGG CGG GCC CTG GGG GTC CTC AGC GTG     48
   Met Val Ala Gly Ser Asp Ala Gly Arg Ala Leu Gly Val Leu Ser Val
   -28         -25                 -20                     -15
   GTC TGC CTG CTG CAC TGC TTT GGT TTC ATC AGC TGT TTT TCC CAA CAA     96
   Val Cys Leu Leu His Cys Phe Gly Phe Ile Ser Cys Phe Ser Gln Gln
           -10                 -5                      1
   ATA TAT GGT GTT GTG TAT GGG AAT GTA ACT TTC CAT GTA CCA AGC AAT    144
   Ile Tyr Gly Val Val Tyr Gly Asn Val Thr Phe His Val Pro Ser Asn
    5                  10                  15                  20
   GTG CCT TTA AAA GAG GTC CTA TGG AAA AAA CAA AAG GAT AAA GTT GCA    192
   Val Pro Leu Lys Glu Val Leu Trp Lys Lys Gln Lys Asp Lys Val Ala
                   25                  30                  35
   GAA CTG GAA AAT TCT GAA TTC AGA GCT TTC TCA TCT TTT AAA AAT AGG    240
   Glu Leu Glu Asn Ser Glu Phe Arg Ala Phe Ser Ser Phe Lys Asn Arg
               40                  45                  50
   GTT TAT TTA GAC ACT GTG TCA GGT AGC CTC ACT ATC TAC AAC TTA ACA    288
   Val Tyr Leu Asp Thr Val Ser Gly Ser Leu Thr Ile Tyr Asn Leu Thr
           55                  60                  65
   TCA TCA GAT GAA GAT GAG TAT GAA ATG GAA TCG CCA AAT ATT ACT GAT    336
   Ser Ser Asp Glu Asp Glu Tyr Glu Met Glu Ser Pro Asn Ile Thr Asp
       70                  75                  80
   ACC ATG AAG TTC TTT CTT TAT GTG CTT GAG TCT CTT CCA TCT CCC ACA    384
   Thr Met Lys Phe Phe Leu Tyr Val Leu Glu Ser Leu Pro Ser Pro Thr
    85                 90                  95                  100
   CTA ACT TGT GCA TTG ACT AAT GGA AGC ATT GAA GTC CAA TGC ATG ATA    432
   Leu Thr Cys Ala Leu Thr Asn Gly Ser Ile Glu Val Gln Cys Met Ile
                   105                 110                 115
   CCA GAG CAT TAC AAC AGC CAT CGA GGA CTT ATA ATG TAC TCA TGG GAT    480
   Pro Glu His Tyr Asn Ser His Arg Gly Leu Ile Met Tyr Ser Trp Asp
               120                 125                 130
   TGT CCT ATG GAG CAA TGT AAA CGT AAC TCA ACC AGT ATA TAT TTT AAG    528
   Cys Pro Met Glu Gln Cys Lys Arg Asn Ser Thr Ser Ile Tyr Phe Lys
           135                 140                 145
   ATG GAA AAT GAT CTT CCA CAA AAA ATA CAG TGT ACT CTT AGC AAT CCA    576
   Met Glu Asn Asp Leu Pro Gln Lys Ile Gln Cys Thr Leu Ser Asn Pro
       150                 155                 160
   TTA TTT AAT ACA ACA TCA TCA ATC ATT TTG ACA ACC TGT ATC CCA AGC    624
   Leu Phe Asn Thr Thr Ser Ser Ile Ile Leu Thr Thr Cys Ile Pro Ser
   165                 170                 175                 180
   AGC GGT CAT TCA AGA CAC AGA TAT GCA CTT ATA CCC ATA CCA TTA GCA    672
   Ser Gly His Ser Arg His Arg Tyr Ala Leu Ile Pro Ile Pro Leu Ala
                   185                 190                 195
   GTA ATT ACA ACA TGT ATT GTG CTG TAT ATG AAT GGT ATG TAT GCT TTT    720
   Val Ile Thr Thr Cys Ile Val Leu Tyr Met Asn Gly Met Tyr Ala Phe
               200                 205                 210
   TAA                                                                723

### (2) INFORMATION FOR SEQ ID NO:4:

(i) SEQUENCE CHARACTERISTICS:
   (A) LENGTH: 240 amino acids
   (B) TYPE: amino acid
   (D) TOPOLOGY: linear
(ii) MOLECULE TYPE: protein
(xi) SEQUENCE DESCRIPTION: SEQ ID NO:4:
   Met Val Ala Gly Ser Asp Ala Gly Arg Ala Leu Gly Val Leu Ser Val
   -28         -25                 -20                 -15
   Val Cys Leu Leu His Cys Phe Gly Phe Ile Ser Cys Phe Ser Gln Gln
           -10                 -5                  1
   Ile Tyr Gly Val Val Tyr Gly Asn Val Thr Phe His Val Pro Ser Asn
   5                   10                  15                  20
   Val Pro Leu Lys Glu Val Leu Trp Lys Lys Gln Lys Asp Lys Val Ala
                   25                  30                  35
   Glu Leu Glu Asn Ser Glu Phe Arg Ala Phe Ser Ser Phe Lys Asn Arg
               40                  45                  50
   Val Tyr Leu Asp Thr Val Ser Gly Ser Leu Thr Ile Tyr Asn Leu Thr
           55                  60                  65
   Ser Ser Asp Glu Asp Glu Tyr Glu Met Glu Ser Pro Asn Ile Thr Asp
       70                  75                  80
   Thr Met Lys Phe Phe Leu Tyr Val Leu Glu Ser Leu Pro Ser Pro Thr
   85                  90                  95                  100
   Leu Thr Cys Ala Leu Thr Asn Gly Ser Ile Glu Val Gln Cys Met Ile
                   105                 110                 115
   Pro Glu His Tyr Asn Ser His Arg Gly Leu Ile Met Tyr Ser Trp Asp
               120                 125                 130
   Cys Pro Met Glu Gln Cys Lys Arg Asn Ser Thr Ser Ile Tyr Phe Lys
           135                 140                 145
   Met Glu Asn Asp Leu Pro Gln Lys Ile Gln Cys Thr Leu Ser Asn Pro
       150                 155                 160
   Leu Phe Asn Thr Thr Ser Ser Ile Ile Leu Thr Thr Cys Ile Pro Ser
   165                 170                 175                 180
   Ser Gly His Ser Arg His Arg Tyr Ala Leu Ile Pro Ile Pro Leu Ala
                   185                 190                 195
   Val Ile Thr Thr Cys Ile Val Leu Tyr Met Asn Gly Met Tyr Ala Phe
               200                 205                 210

### (2) INFORMATION FOR SEQ ID NO:5:

(i) SEQUENCE CHARACTERISTICS:
   (A) LENGTH: 1056 base pairs
   (B) TYPE: nucleic acid
   (C) STRANDEDNESS: single
   (D) TOPOLOGY: linear
(ii) MOLECULE TYPE: cDNA
(ix) FEATURE:
   (A) NAME/KEY: CDS
   (B) LOCATION: 1..1053
(ix) FEATURE:
   (A) NAME/KEY: sig_peptide
   (B) LOCATION: 1..72
(ix) FEATURE:
   (A) NAME/KEY: mat_peptide
   (B) LOCATION: 73..1053
(ix) FEATURE:
   (A) NAME/KEY: misc_feature
   (B) LOCATION: 1..1053
   (D) OTHER INFORMATION: /note= "Human CD2"
(ix) FEATURE:
   (A) NAME/KEY: misc_feature
   (B) LOCATION: 628..702
   (D) OTHER INFORMATION: /note= "Transmembrane domain"
(xi) SEQUENCE DESCRIPTION: SEQ ID NO:5:
   ATG AGC TTT CCA TGT AAA TTT GTA GCC AGC TTC CTT CTG ATT TTC AAT      48
   Met Ser Phe Pro Cys Lys Phe Val Ala Ser Phe Leu Leu Ile Phe Asn
   -24             -20                 -15                 -10
   GTT TCT TCC AAA GGT GCA GTC TCC AAA GAG ATT ACG AAT GCC TTG GAA      96
   Val Ser Ser Lys Gly Ala Val Ser Lys Glu Ile Thr Asn Ala Leu Glu
               -5                  1               5
   ACC TGG GGT GCC TTG GGT CAG GAC ATC AAC TTG GAC ATT CCT AGT TTT     144
   Thr Trp Gly Ala Leu Gly Gln Asp Ile Asn Leu Asp Ile Pro Ser Phe
        10                  15                  20
   CAA ATG AGT GAT GAT ATT GAC GAT ATA AAA TGG GAA AAA ACT TCA GAC     192
   Gln Met Ser Asp Asp Ile Asp Asp Ile Lys Trp Glu Lys Thr Ser Asp
    25                  30                  35                  40
   AAG AAA AAG ATT GCA CAA TTC AGA AAA GAG AAA GAG ACT TTC AAG GAA     240
   Lys Lys Lys Ile Ala Gln Phe Arg Lys Glu Lys Glu Thr Phe Lys Glu
                    45                  50                  55
   AAA GAT ACA TAT AAG CTA TTT AAA AAT GGA ACT CTG AAA ATT AAG CAT     288
   Lys Asp Thr Tyr Lys Leu Phe Lys Asn Gly Thr Leu Lys Ile Lys His
                60                  65                  70
   CTG AAG ACC GAT GAT CAG GAT ATC TAC AAG GTA TCA ATA TAT GAT ACA     336
   Leu Lys Thr Asp Asp Gln Asp Ile Tyr Lys Val Ser Ile Tyr Asp Thr
            75                  80                  85
   AAA GGA AAA AAT GTG TTG GAA AAA ATA TTT GAT TTG AAG ATT CAA GAG     384
   Lys Gly Lys Asn Val Leu Glu Lys Ile Phe Asp Leu Lys Ile Gln Glu
        90                  95                 100
   AGG GTC TCA AAA CCA AAG ATC TCC TGG ACT TGT ATC AAC ACA ACC CTG     432
   Arg Val Ser Lys Pro Lys Ile Ser Trp Thr Cys Ile Asn Thr Thr Leu
   105                 110                 115                 120
   ACC TGT GAG GTA ATG AAT GGA ACT GAC CCC GAA TTA AAC CTG TAT CAA     480
   Thr Cys Glu Val Met Asn Gly Thr Asp Pro Glu Leu Asn Leu Tyr Gln
                   125                 130                 135
   GAT GGG AAA CAT CTA AAA CTT TCT CAG AGG GTC ATC ACA CAC AAG TGG     528
   Asp Gly Lys His Leu Lys Leu Ser Gln Arg Val Ile Thr His Lys Trp
               140                 145                 150
   ACC ACC AGC CTG AGT GCA AAA TTC AAG TGC ACA GCA GGG AAC AAA GTC     576
   Thr Thr Ser Leu Ser Ala Lys Phe Lys Cys Thr Ala Gly Asn Lys Val
           155                 160                 165
   AGC AAG GAA TCC AGT GTC GAG CCT GTC AGC TGT CCA GAG AAA GGT CTG     624
   Ser Lys Glu Ser Ser Val Glu Pro Val Ser Cys Pro Glu Lys Gly Leu
       170                 175                 180
   GAC ATC TAT CTC ATC ATT GGC ATA TGT GGA GGA GGC AGC CTC TTG ATG     672
   Asp Ile Tyr Leu Ile Ile Gly Ile Cys Gly Gly Gly Ser Leu Leu Met
   185                 190                 195                 200
   GTC TTT GTG GCA CTG CTC GTT TTC TAT ATC ACC AAA AGG AAA AAA CAG     720
   Val Phe Val Ala Leu Leu Val Phe Tyr Ile Thr Lys Arg Lys Lys Gln
                   205                 210                 215
   AGG AGT CGG AGA AAT GAT GAG GAG CTG GAG ACA AGA GCC CAC AGA GTA     768
   Arg Ser Arg Arg Asn Asp Glu Glu Leu Glu Thr Arg Ala His Arg Val
               220                 225                 230
   GCT ACT GAA GAA AGG GGC CGG AAG CCC CAC CAA ATT CCA GCT TCA ACC     816
   Ala Thr Glu Glu Arg Gly Arg Lys Pro His Gln Ile Pro Ala Ser Thr
       235                 240                 245
   CCT CAG AAT CCA GCA ACT TCC CAA CAT CCT CCT CCA CCA CCT GGT CAT     864
   Pro Gln Asn Pro Ala Thr Ser Gln His Pro Pro Pro Pro Pro Gly His
       250                 255                 260
   CGT TCC CAG GCA CCT AGT CAT CGT CCC CCG CCT CCT GGA CAC CGT GTT     912
   Arg Ser Gln Ala Pro Ser His Arg Pro Pro Pro Pro Gly His Arg Val
   265                 270                 275                 280
   CAG CAC CAG CCT CAG AAG AGG CCT CCT GCT CCG TCG GGC ACA CAA GTT     960
   Gln His Gln Pro Gln Lys Arg Pro Pro Ala Pro Ser Gly Thr Gln Val
                   285                 290                 295
   CAC CAG CAG AAA GGC CCG CCC CTC CCC AGA CCT CGA GTT CAG CCA AAA    1008
   His Gln Gln Lys Gly Pro Pro Leu Pro Arg Pro Arg Val Gln Pro Lys
               300                 305                 310
   CCT CCC CAT GGG GCA GCA GAA AAC TCA TTG TCC CCT TCC TCT AAT        1053
   Pro Pro His Gly Ala Ala Glu Asn Ser Leu Ser Pro Ser Ser Asn
       315                 320                 325
   TAA                                                                1056

### (2) INFORMATION FOR SEQ ID NO:6:

(i) SEQUENCE CHARACTERISTICS:
   (A) LENGTH: 351 amino acids
   (B) TYPE: amino acid
   (D) TOPOLOGY: linear
(ii) MOLECULE TYPE: protein
(xi) SEQUENCE DESCRIPTION: SEQ ID NO:6:
   Met Ser Phe Pro Cys Lys Phe Val Ala Ser Phe Leu Leu Ile Phe Asn
   -24             -20                 -15                 -10
   Val Ser Ser Lys Gly Ala Val Ser Lys Glu Ile Thr Asn Ala Leu Glu
               -5                  1               5
   Thr Trp Gly Ala Leu Gly Gln Asp Ile Asn Leu Asp Ile Pro Ser Phe
       10                  15                  20
   Gln Met Ser Asp Asp Ile Asp Asp Ile Lys Trp Glu Lys Thr Ser Asp
   25                  30                  35                  40
   Lys Lys Lys Ile Ala Gln Phe Arg Lys Glu Lys Glu Thr Phe Lys Glu
                   45                  50                  55
   Lys Asp Thr Tyr Lys Leu Phe Lys Asn Gly Thr Leu Lys Ile Lys His
               60                  65                  70
   Leu Lys Thr Asp Asp Gln Asp Ile Tyr Lys Val Ser Ile Tyr Asp Thr
           75                  80                  85
   Lys Gly Lys Asn Val Leu Glu Lys Ile Phe Asp Leu Lys Ile Gln Glu
       90                  95                  100
   Arg Val Ser Lys Pro Lys Ile Ser Trp Thr Cys Ile Asn Thr Thr Leu
   105                 110                 115                 120
   Thr Cys Glu Val Met Asn Gly Thr Asp Pro Glu Leu Asn Leu Tyr Gln
                   125                 130                 135
   Asp Gly Lys His Leu Lys Leu Ser Gln Arg Val Ile Thr His Lys Trp
               140                 145                 150
   Thr Thr Ser Leu Ser Ala Lys Phe Lys Cys Thr Ala Gly Asn Lys Val
           155                 160                 165
   Ser Lys Glu Ser Ser Val Glu Pro Val Ser Cys Pro Glu Lys Gly Leu
       170                 175                 180
   Asp Ile Tyr Leu Ile Ile Gly Ile Cys Gly Gly Gly Ser Leu Leu Met
   185                 190                 195                 200
   Val Phe Val Ala Leu Leu Val Phe Tyr Ile Thr Lys Arg Lys Lys Gln
                   205                 210                 215
   Arg Ser Arg Arg Asn Asp Glu Glu Leu Glu Thr Arg Ala His Arg Val
               220                 225                 230
   Ala Thr Glu Glu Arg Gly Arg Lys Pro His Gln Ile Pro Ala Ser Thr
           235                 240                 245
   Pro Gln Asn Pro Ala Thr Ser Gln His Pro Pro Pro Pro Pro Gly His
       250                 255                 260
   Arg Ser Gln Ala Pro Ser His Arg Pro Pro Pro Pro Gly His Arg Val
   265                 270                 275                 280
   Gln His Gln Pro Gln Lys Arg Pro Pro Ala Pro Ser Gly Thr Gln Val
                   285                 290                 295
   His Gln Gln Lys Gly Pro Pro Leu Pro Arg Pro Arg Val Gln Pro Lys
               300                 305                 310
   Pro Pro His Gly Ala Ala Glu Asn Ser Leu Ser Pro Ser Ser Asn
           315                 320                 325

### (2) INFORMATION FOR SEQ ID NO:7:

(i) SEQUENCE CHARACTERISTICS:
   (A) LENGTH: 1050 base pairs
   (B) TYPE: nucleic acid
   (C) STRANDEDNESS: single
   (D) TOPOLOGY: linear
(ii) MOLECULE TYPE: cDNA
(ix) FEATURE:
   (A) NAME/KEY: CDS
   (B) LOCATION: 1..1041
(ix) FEATURE:
   (A) NAME/KEY: sig_peptide
   (B) LOCATION: 1..84
(ix) FEATURE:
   (A) NAME/KEY: mat_peptide
   (B) LOCATION: 85..1041
(ix) FEATURE:
   (A) NAME/KEY: misc_feature
   (B) LOCATION: 85..1041
   (D) OTHER INFORMATION: /note- "LFA3TIP"
(ix) FEATURE:
   (A) NAME/KEY: misc_feature
   (B) LOCATION: 360..361
   (D) OTHER INFORMATION: /note- "LFA-3/IgG fusion point"
(xi) SEQUENCE DESCRIPTION: SEQ ID NO:7:
   ATG GTT GCT GGG AGC GAC GCG GGG CGG GCC CTG GGG GTC CTC AGC GTG      48
   Met Val Ala Gly Ser Asp Ala Gly Arg Ala Leu Gly Val Leu Ser Val
   -28         -25                 -20                 -15
   GTC TGC CTG CTG CAC TGC TTT GGT TTC ATC AGC TGT TTT TCC CAA CAA      96
   Val Cys Leu Leu His Cys Phe Gly Phe Ile Ser Cys Phe Ser Gln Gln
           -10                  -5                   1
   ATA TAT GGT GTT GTG TAT GGG AAT GTA ACT TTC CAT GTA CCA AGC AAT     144
   Ile Tyr Gly Val Val Tyr Gly Asn Val Thr Phe His Val Pro Ser Asn
     5                  10                  15                  20
   GTG CCT TTA AAA GAG GTC CTA TGG AAA AAA CAA AAG GAT AAA GTT GCA     192
   Val Pro Leu Lys Glu Val Leu Trp Lys Lys Gln Lys Asp Lys Val Ala
                    25                  30                  35
   GAA CTG GAA AAT TCT GAA TTC AGA GCT TTC TCA TCT TTT AAA AAT AGG     240
   Glu Leu Glu Asn Ser Glu Phe Arg Ala Phe Ser Ser Phe Lys Asn Arg
                40                  45                  50
   GTT TAT TTA GAC ACT GTG TCA GGT AGC CTC ACT ATC TAC AAC TTA ACA     288
   Val Tyr Leu Asp Thr Val Ser Gly Ser Leu Thr Ile Tyr Asn Leu Thr
            55                  60                  65
   TCA TCA GAT GAA GAT GAG TAT GAA ATG GAA TCG CCA AAT ATT ACT GAT     336
   Ser Ser Asp Glu Asp Glu Tyr Glu Met Glu Ser Pro Asn Ile Thr Asp
        70                  75                  80
   ACC ATG AAG TTC TTT CTT TAT GTC GAC AAA ACT CAC ACA TGC CCA CCG     384
   Thr Met Lys Phe Phe Leu Tyr Val Asp Lys Thr His Thr Cys Pro Pro
    85                  90                  95                 100
   TGC CCA GCA CCT GAA CTC CTG GGG GGA CCG TCA GTC TTC CTC TTC CCC     432
   Cys Pro Ala Pro Glu Leu Leu Gly Gly Pro Ser Val Phe Leu Phe Pro
                   105                 110                 115
   CCA AAA CCC AAG GAC ACC CTC ATG ATC TCC CGG ACC CCT GAG GTC ACA     480
   Pro Lys Pro Lys Asp Thr Leu Met Ile Ser Arg Thr Pro Glu Val Thr
               120                 125                 130
   TGC GTG GTG GTG GAC GTG AGC CAC GAA GAC CCT GAG GTC AAG TTC AAC         528
   Cys Val Val Val Asp Val Ser His Glu Asp Pro Glu Val Lys Phe Asn
           135                 140                 145
   TGG TAC GTG GAC GGC GTG GAG GTG CAT AAT GCC AAG ACA AAG CCG CGG         576
   Trp Tyr Val Asp Gly Val Glu Val His Asn Ala Lys Thr Lys Pro Arg
       150                 155                 160
   GAG GAG CAG TAC AAC AGC ACG TAC CGG GTG GTC AGC GTC CTC ACC GTC         624
   Glu Glu Gln Tyr Asn Ser Thr Tyr Arg Val Val Ser Val Leu Thr Val
   165                 170                 175                 180
   CTG CAC CAG GAC TGG CTG AAT GGC AAG GAG TAC AAG TGC AAG GTC TCC         672
   Leu His Gln Asp Trp Leu Asn Gly Lys Glu Tyr Lys Cys Lys Val Ser
                   185                 190                 195
   AAC AAA GCC CTC CCA GCC CCC ATC GAG AAA ACC ATC TCC AAA GCC AAA         720
   Asn Lys Ala Leu Pro Ala Pro Ile Glu Lys Thr Ile Ser Lys Ala Lys
               200                 205                 210
   GGG CAG CCC CGA GAA CCA CAG GTG TAC ACC CTG CCC CCA TCC CGG GAT         768
   Gly Gln Pro Arg Glu Pro Gln Val Tyr Thr Leu Pro Pro Ser Arg Asp
           215                 220                 225
   GAG CTG ACC AAG AAC CAG GTC AGC CTG ACC TGC CTG GTC AAA GGC TTC         816
   Glu Leu Thr Lys Asn Gln Val Ser Leu Thr Cys Leu Val Lys Gly Phe
       230                 235                 240
   TAT CCC AGC GAC ATC GCC GTG GAG TGG GAG AGC AAT GGG CAG CCG GAG         864
   Tyr Pro Ser Asp Ile Ala Val Glu Trp Glu Ser Asn Gly Gln Pro Glu
   245                 250                 255                 260
   AAC AAC TAC AAG ACC ACG CCT CCC GTG CTG GAC TCC GAC GGC TCC TTC         912
   Asn Asn Tyr Lys Thr Thr Pro Pro Val Leu Asp Ser Asp Gly Ser Phe
                   265                 270                 275
   TTC CTC TAC AGC AAG CTC ACC GTG GAC AAG AGC AGG TGG CAG CAG GGG         960
   Phe Leu Tyr Ser Lys Leu Thr Val Asp Lys Ser Arg Trp Gln Gln Gly
               280                 285                 290
   AAC GTC TTC TCA TGC TCC GTG ATG CAT GAG GCT CTG CAC AAC CAC TAC        1008
   Asn Val Phe Ser Cys Ser Val Met His Glu Ala Leu His Asn His Tyr
           295                 300                 305
   ACG CAG AAG AGC CTC TCC CTG TCT CCG GGT AAA TGAGTGCGG                  1050
   Thr Gln Lys Ser Leu Ser Leu Ser Pro Gly Lys
       310                 315

### (2) INFORMATION FOR SEQ ID NO:8:

(i) SEQUENCE CHARACTERISTICS:
   (A) LENGTH: 347 amino acids
   (B) TYPE: amino acid
   (D) TOPOLOGY: linear
(ii) MOLECULE TYPE: protein
(xi) SEQUENCE DESCRIPTION: SEQ ID NO:8:
   Met Val Ala Gly Ser Asp Ala Gly Arg Ala Leu Gly Val Leu Ser Val
   -28         -25                 -20                 -15
   Val Cys Leu Leu His Cys Phe Gly Phe Ile Ser Cys Phe Ser Gln Gln
           -10                 -5                   1
   Ile Tyr Gly Val Val Tyr Gly Asn Val Thr Phe His Val Pro Ser Asn
    5                  10                  15                  20
   Val Pro Leu Lys Glu Val Leu Trp Lys Lys Gln Lys Asp Lys Val Ala
                   25                  30                  35
   Glu Leu Glu Asn Ser Glu Phe Arg Ala Phe Ser Ser Phe Lys Asn Arg
               40                  45                  50
   Val Tyr Leu Asp Thr Val Ser Gly Ser Leu Thr Ile Tyr Asn Leu Thr
           55                  60                  65
   Ser Ser Asp Glu Asp Glu Tyr Glu Met Glu Ser Pro Asn Ile Thr Asp
       70                  75                  80
   Thr Met Lys Phe Phe Leu Tyr Val Asp Lys Thr His Thr Cys Pro Pro
   85                  90                  95                  100
   Cys Pro Ala Pro Glu Leu Leu Gly Gly Pro Ser Val Phe Leu Phe Pro
                   105                 110                 115
   Pro Lys Pro Lys Asp Thr Leu Met Ile Ser Arg Thr Pro Glu Val Thr
               120                 125                 130
   Cys Val Val Val Asp Val Ser His Glu Asp Pro Glu Val Lys Phe Asn
           135                 140                 145
   Trp Tyr Val Asp Gly Val Glu Val His Asn Ala Lys Thr Lys Pro Arg
       150                 155                 160
   Glu Glu Gln Tyr Asn Ser Thr Tyr Arg Val Val Ser Val Leu Thr Val
   165                 170                 175                 180
   Leu His Gln Asp Trp Leu Asn Gly Lys Glu Tyr Lys Cys Lys Val Ser
                   185                 190                 195
   Asn Lys Ala Leu Pro Ala Pro Ile Glu Lys Thr Ile Ser Lys Ala Lys
               200                 205                 210
   Gly Gln Pro Arg Glu Pro Gln Val Tyr Thr Leu Pro Pro Ser Arg Asp
           215                 220                 225
   Glu Leu Thr Lys Asn Gln Val Ser Leu Thr Cys Leu Val Lys Gly Phe
       230                 235                 240
   Tyr Pro Ser Asp Ile Ala Val Glu Trp Glu Ser Asn Gly Gln Pro Glu
   245                 250                 255                 260
   Asn Asn Tyr Lys Thr Thr Pro Pro Val Leu Asp Ser Asp Gly Ser Phe
                   265                 270                 275
   Phe Leu Tyr Ser Lys Leu Thr Val Asp Lys Ser Arg Trp Gln Gln Gly
               280                 285                 290
   Asn Val Phe Ser Cys Ser Val Met His Glu Ala Leu His Asn His Tyr
           295                 300                 305
   Thr Gln Lys Ser Leu Ser Leu Ser Pro Gly Lys
       310                 315